# EUROPEAN PATENT SPECIFICATION

(11) **EP 1 827 250 B1**
(45) Date of publication and mention of the grant of the patent: **16.05.2018**
(21) Application number: 05794832.5
(22) Date of filing: 31.08.2005
(51) Int. Cl.: A61B 17/12

(54) **DEVICE FOR TREATING AN ANEURYSM**
VORRICHTUNG ZUR BEHANDLUNG VON ANEURYSMEN
DISPOSITIF DE TRAITEMENT D'ANEVRISME

(30) Priority: 31.08.2004 US 605805 P; 31.08.2004 US 606260 P; 17.03.2005 US 662666 P
(43) Date of publication of application: 05.09.2007
(73) Proprietor: Cook Medical Technologies LLC, Bloomington, IN 47404 (US)
(72) Inventor: BATES, Brian, L., Bloomington, IN 47401 (US); CONDER, Andrew, W., Bloomington, IN 47401 (US); BUTLER, Gary, L., Bloomington, IN 47404 (US)
(74) Representative: Garratt, Peter Douglas
(86) International application number: PCT/US2005/031277
(87) International publication number: WO 2006/026744

(56) References cited:
- EP-A1- 1 188 414
- WO-A-02/071977
- WO-A1-00/32112
- WO-A1-94/06460
- WO-A1-99/05977
- WO-A1-03/090837
- US-A1- 2002 165 572
- US-A1- 2003 028 209
- US-B1- 6 168 622
- US-B1- 6 245 090

## Description

### BACKGROUND

The present invention relates generally to the treatment of aneurysms and more particularly, to a basket implanted into an aneurysm using an intravascular technique. Aneurysms are dilations that are caused from weakening of a blood vessel wall. The dilation is produced by the pressure exerted by normal blood flow, which causes the weakened segment of the blood vessel, such as an artery or vein, to swell. In some types of aneurysms, such as intracranial aneurysms, this swelling may result in a balloon-like polyp (sometimes referred to as berry aneurysms). In other cases the swelling causes a circumscribed bulge in the blood vessel, as is the case with aortic aneurysms. Continued growth and/or eventual rupture of the ballooned arterial wall can have devastating results for patients. Consequently, unruptured aneurysms are usually treated to prevent hemorage, while ruptured aneurysms are typically treated to avert rerupture and additional concomitant damage.

There are presently three general methods of treating intracranial aneurysms. In general, these methods can be described as extravascular approaches, extra-intravascular approaches and intravascular approaches. The traditional method of treatment is the extravascular approach, which is a surgical procedure commonly known as a craniotomy. In this highly invasive procedure, a portion of the skullcap is removed and the brain is retracted to allow the surgeon to locate the aneurysm. An arachnoid dissection is then performed around the neck of the aneurysm. Next, the aneurysm is closed-off from the main vessel. Closing-off the aneurysm is usually achieved by clipping the neck of the aneurysm, performing a suture-ligation of the neck, or wrapping the entire aneurysm. Unfortunately, as one might guess, this technique poses significant risk for patients and requires extended recovery times.

The extra-intravascular approach is also a surgical technique that exposes the aneurysm. Once the aneurysm is exposed, the wall of the aneurysm is perforated from the exterior of the aneurysm and various techniques are used to occlude the aneurysm. Extra-intravascular techniques include procedures such as electrothrombosis and isobutyl-cyanoacrylate embolization (IBCA). Electrothrombosis involves the surgical insertion of a positively charged electrode into the interior of the aneurysm. The positive charge attracts white blood cells, red blood cells, platelets and fibrinogen, all of which are usually negatively charged in vivo, forming a thrombic mass in the aneurysm. Thereafter, the tip is removed.

Injection of IBCA into an aneurysm, as previously mentioned, is another method of extra-intravascular aneurysm treatment. In this procedure, the aneurysm is punctured with a small needle and IBCA is injected, which serves to form an occlusion, since the IBCA polymerizes rapidly on contact with blood to form a firm mass. However, if the IBCA leaks out of the aneurysm and is allowed to enter the artery it can have negative consequences for the patient. Whether elctrothrombosis, IBCA or some other extra-intravascular technique is employed, all of these procedures require invasive surgical procedures as described above.

There are a number of intravascular approaches, in which the interior of the aneurysm is accessed using a microcatheter. Typically, these methods of treatment involve filling the aneurysm to inhibit blood flow into the aneurismal sac. Reduced blood flow usually correlates to a reduction in aneurismal pressure and, hence, a reduction in aneurysm growth and the likelihood of rupture. Access to the vascular system and subsequent entry into the cranial aneurysm is usually achieved using a microcatheter in the Seldinger technique.

One type of intravascular treatment is balloon embolization. In this procedure a balloon is attached to the end of the microcatheter, introduced into the aneurysm, inflated, and detached, leaving it to occlude the sac and neck while preserving the parent artery. While intravascular balloon embolization of berry aneurysms is sometimes an effective method of treatment, especially where an extravascular surgical approach may be difficult, inflation of a balloon within the aneurysm can cause the aneurysm to rupture due to possible over-distention and traction produced while detaching the balloon. Ideally, the embolizing agent should adapt itself to the irregular internal shape of the aneurysm. However in contrast, balloon embolization requires the aneurysm instead to conform to the shape of the balloon. While remedial procedures exist for treating a ruptured aneurysm during classical extravascular surgery, no satisfactory methodology exists if the aneurysm breaks during an intravascular balloon embolization.

Another common intravascular method of treatment is microcoil thrombosis, which employs a microcatheter to reach the aneurysm in the same manner as described for balloon embolization. However, instead of delivering a balloon into the aneurysm, this procedure delivers one or more metal coils to inhibit blood flow into the aneurysm and produce formation of a thrombic mass. The number of coils used may be dependant upon the size and shape of the aneurysm. Thus, this technique may require multiple metal coils. As a result, this technique may be expensive and time consuming.

One disadvantage of many of the current treatments for intracranial aneurysms is that they may result in mass effect. Mass effect may occur when the aneurysm is filled with a solid mass. As a result, the aneurysm is no longer malleable or pliable, like the normal tissue. As a result, the aneurysm may exert pressure against the surrounding tissue. The resulting symptoms can include dizziness, vomiting, and headaches. The solid mass of the aneurysm may also apply pressure to the surrounding tissues, where this pressure is amplified when the person moves his or her body during normal or increased activity.

Unfortunately, some of these treatment methods may only be effective for a short period of time, due to reformation of the aneurysm. Reformation of an aneurysm can occur if blood gains access to the treated aneurysm, for example by working its way along the edges of the micro-coil/thrombic mass. If the blood is able to gain access to the treated aneurysm, the resulting blood pressure will cause additional swelling of the aneurysm wall, leading to reformation of the aneurysm. Ideally, the treatment for intracranial aneurysms would be less expensive, require fewer manipulations during treatment, have fewer side-effects and have fewer complications. In addition, the treatment should decrease the potential for reformation of the aneurysm.

Reference is directed to US 6,168,622 which discloses an aneurysm occlusion device having a bulbous body portion and an anchor. The body portion is sized to be received within an aneurysm and the anchor is sized and shaped to engage an interior surface of the vessel's wall. The body portion and the anchor are integrally formed of a resilient fabric permitting the occlusion device to be collapsed for deployment and resiliently self-expanded to include the aneurysm.

### BRIEF SUMMARY

The present invention is set forth in the appended claims. In one aspect, there is described an aneurysm basket or sealing device that has a basket wall extending between a proximal basket end and a distal basket end, where the basket wall encloses a cavity and the proximal basket end comprises a ring. The aneurysm basket also includes a basket neck region extending between the proximal basket end and a distal neck end, as well as a spherical region extending between the distal neck end and the distal basket end. Additionally the aneurysm basket includes a patch circumscribed by and attached to the ring, where the patch comprises a pusher wire access point. The aneurysm basket also has a plurality of wires extending between a plurality of proximal wire ends and a plurality of distal wire ends, where the plurality of wires comprise the basket wall, the plurality of proximal wire ends comprise the proximal basket end, and the plurality of distal wire ends extend from the distal basket end into the cavity. Furthermore, the aneurysm basket includes a cannula extending between a distal cannula end and a proximal cannula end, where the cannula fastens together the plurality of distal wire ends and the cannula further comprising an attachment. The aneurysm basket also has a pusher wire, where the pusher wire extends through the pusher wire access point and the attachment connects the pusher wire and the cannula.

In another aspect, there is described an aneurysm basket that includes a basket wall extending between a proximal basket end and a distal basket end, where the basket wall encloses a cavity and the proximal basket end comprises a ring. The aneurysm basket also includes a basket neck region extending between the proximal basket end and a distal neck end. In addition, the aneurysm basket possesses a spherical region extending between the distal neck end and the distal basket end. Furthermore, the aneurysm basket includes a plurality of wires, where the plurality of wires comprise a plurality of proximal wire ends, the plurality of wires comprise the basket wall, and the plurality of proximal wire ends comprise the proximal basket end.

In a further aspect, there is described an aneurysm basket that includes a basket wall extending between a proximal basket end and a distal basket end, where the basket wall encloses a cavity and the proximal basket end comprises a ring. The aneurysm basket also includes a basket neck region extending between the proximal basket end and a distal neck end. In addition, the aneurysm basket possesses a spherical region extending between the distal neck end and the distal basket end. The aneurysm basket also comprises a patch circumscribed by and attached to the ring. Furthermore, the aneurysm basket includes a plurality of wires, where the plurality of wires include a plurality of proximal wire ends, the plurality of wires comprise the basket wall, and the plurality proximal wire ends comprise the proximal basket end. The plurality of wires extend between the plurality of proximal wire ends and a plurality of distal wire ends, such that the plurality of distal wire ends extend from the distal basket end into the cavity. The aneurysm basket also includes a cannula extending between a distal cannula end and a proximal cannula end, where the cannula fastens together the plurality of distal wire ends and the cannula also includes an attachment. Furthermore, the aneurysm basket includes a pusher wire access point, the pusher wire access point being located in the patch, and a pusher wire. The pusher wire extends through the pusher wire access point and the pusher wire is connected to the cannula by way of the attachment, where the attachment is selectively detachable.

In an additional aspect, there is described a device for treating an aneurysm, comprising an anchor member expandable between a collapsed state and an expanded state, the anchor member being adapted to be implanted intravascularly into an aneurysm. The anchor member extends between a proximal anchor end and a distal anchor end. The device also includes a patch made of a covering material attached to the proximal anchor end, where the patch is adapted to cover a neck of the aneurysm and the anchor member secures the patch to the neck of the aneurysm thereby restricting blood flow into the aneurysm.

In another aspect, there is described a method of treating an aneurysm, comprising securing a patch of covering material to an anchor member. The method also includes delivering the patch and the anchor member intravascularly to an aneurysm. The method further involves inserting the anchor member into at least a neck of the aneurysm, where the anchor member is secured within the aneurysm and the patch covers the neck of the aneurysm thereby restricting blood flow into the aneurysm.

In an additional aspect, there is described a method of treating an aneurysm, comprising securing a patch of covering material to an anchor member. The method also includes delivering the patch and the anchor member intravascularly to an aneurysm. The method further involves inserting the anchor member into at least a neck of the aneurysm, where the anchor member is secured within the aneurysm and the patch covers the neck of the aneurysm thereby restricting blood flow into the aneurysm. In this aspect of the invention, the patch and the anchor member are delivered intravascularly to the aneurysm by way of a microcatheter, where the microcatheter extends between a microcatheter distal end and a microcatheter proximal end. The microcatheter defines a lumen therein and the microcatheter contains a pusher member that is located within the lumen. In addition, the pusher member is situated proximal to the patch, such that the anchor member is slidably movable within the microcatheter. The patch and the anchor member are located within the lumen of the microcatheter and are situated between the microcatheter distal end and the pusher member. The patch and the anchor member are expelled from the microcatheter distal end by sliding the pusher member toward the microcatheter distal end.

In a further aspect, there is described an aneurysm basket comprising a basket wall extending between a proximal basket end and a distal basket end, where the basket wall encloses a cavity and the proximal basket end comprises a ring. Additionally, the aneurysm basket includes a basket neck region extending between the proximal basket end and a distal neck end. The aneurysm basket also possesses a spherical region extending between the distal neck end and the distal basket end. Furthermore, the aneurysm basket includes a patch circumscribed by and attached to the ring, where the patch comprises a pusher wire access point, the patch comprises small intestine submucosa, and the patch is attached to the ring by sutures. The aneurysm basket also has a plurality of wires extending between a plurality of proximal wire ends and a plurality of distal wire ends, where the plurality of wires comprise the basket wall, the proximal wire ends comprise the proximal basket end, the distal wire ends extend from the distal basket end into the cavity, and the plurality of wires are woven to provide a woven basket wall. In addition, the aneurysm basket possesses a releasable member attached to the distal wire ends and located in the cavity, where the releasable member comprises an electrolytic mechanism thereby being selectively detachable. Furthermore, the aneurysm basket includes a pusher wire extending through the pusher wire access point and connected to the releasable member, where the pusher wire is selectively detachable from the distal wire ends.

In another aspect, there is described an aneurysm insert consisting of a body: The body has an insert neck region extending between a proximal insert end and a distal neck end, where the insert neck region is adapted to be inserted into a neck region of an aneurysm. The body also has an insert spherical region extending between the distal neck end and a distal insert end. The insert spherical region is adapted to be inserted into a spherical interior region of an aneurysm, where at least a portion of the body is a compressible material.

A further aspect described is a sealing device for sealing a dilatation area of a body vessel caused by an aneurysm. The sealing device comprises a basket and a base attached to the basket. The basket has an opening formed thereon defining a sealing lip. The basket is configured to be introduced into the aneurysm to maintain position of the sealing device at the dilatation area of the body vessel. The base is attached to the sealing lip closing the opening. The base has connective tissue disposed thereon for tissue repair at the dilatation area of the body vessel. The connective tissue is configured to contact the body vessel to subsequently seal the dilatation area.

In another aspect, the basket is woven and configured to be collapsible and expandable. The base comprises struts attached to the sealing lip and the struts extend inwardly to a common point to collapse and expand the sealing device. In this embodiment, the connective tissue is disposed on the struts for tissue repair at the dilatation area.

In an additional aspect, a sealing apparatus for sealing a dilatation area of a body vessel from an aneurysm is provided. The sealing apparatus comprises an introducer sheath, a catheter to be passed through the introducer sheath, and a pusher wire for advancing the sealing device through the catheter. The catheter has proximal and distal ends and is configured to pass through the introducer sheath to position the catheter in the body vessel. The catheter has a hub adjacent the proximal end through which the sealing device is loaded for deployment in the body vessel.

In yet another aspect, a method of sealing a dilatation area of a body vessel from an aneurysm is provided. The method comprises percutaneously introducing the sealing device in a body vessel for sealing a dilatation area of the body vessel. The method further includes disposing the basket of the sealing device in the aneurysm to maintain position of the sealing device at the dilatation area of the body vessel and contacting the connective tissue with the body vessel to seal the dilatation area. The method further includes releasing the sealing device at the dilatation area for tissue repair.

In another aspect , a sealing apparatus for sealing a dilatation area of a body vessel from an aneurysm is disclosed. The sealing apparatus comprises a sealing device including a basket having an opening formed thereon defining a sealing lip. The basket is configured to be introduced in the aneurysm to maintain the position of the sealing device at the dilatation area of the body vessel. The sealing device further comprises a base attached to the sealing lip closing the opening. Furthermore, the base has connective tissue disposed thereon for tissue repair at the dilatation area of the body vessel, where the connective tissue is configured to contact the body vessel to seal the dilatation area. In addition, the sealing apparatus also comprises an introducer sheath through which the sealing device is disposed for percutaneous insertion into a body vessel, as well as a catheter having both a proximal and a distal end. The catheter is configured to be passed through the introducer sheath to position the catheter in the body vessel and it includes a hub adjacent the proximal end. The sealing apparatus also includes a pusher wire for advancing the sealing device and adjusting the placement thereof during deployment into the body vessel.

In a further aspect, an aneurysm insert is adapted for intravascular delivery to the interior of an aneurysm. The insert comprises a structure that in a rest state intrinsically defines an exterior spheroidal surface. In addition, the structure is compressible to enable passage through a neck of the aneurysm.

In another aspect, an aneurysm insert is adapted for intravascular delivery to the interior of an aneurysm. The insert comprises a structure that in a rest state intrinsically defines an exterior spheroidal surface. In addition, the structure is compressible to enable passage through a neck of the aneurysm and the structure is resiliently compressible from the rest state.

In a further aspect, an aneurysm insert is adapted for intravascular delivery to the interior of an aneurysm. The insert comprises a structure that in a rest state intrinsically defines an exterior spheroidal surface. In addition, the structure is compressible to enable passage through a neck of the aneurysm and the structure is adapted through shape memory to return to the rest state after compression.

In an additional aspect, an aneurysm insert is adapted for intravascular delivery to the interior of an aneurysm. The insert comprises a structure that in a rest state intrinsically defines an exterior spheroidal surface. In addition, the structure is compressible to enable passage through a neck of the aneurysm and the structure is adapted through mechanical biasing to return to the rest state after compression.

In a further aspect, an aneurysm insert is adapted for intravascular delivery to the interior of an aneurysm. The insert comprises a structure that in a rest state intrinsically defines an exterior spheroidal surface. In addition, the structure is compressible to enable passage through a neck of the aneurysm and the structure comprises a spheroidal wire basket.

In another aspect, an aneurysm insert is adapted for intravascular delivery to the interior of an aneurysm. The insert comprises a structure that in a rest state intrinsically defines an exterior spheroidal surface. In addition, the structure is compressible to enable passage through a neck of the aneurysm and the structure comprises a spheroidal wire basket, where the wire basket comprises two mutually orthogonal wire paths.

In a further aspect, an aneurysm insert is adapted for intravascular delivery to the interior of an aneurysm. The insert comprises a structure that in a rest state intrinsically defines an exterior spheroidal surface. In addition, the structure is compressible to enable passage through a neck of the aneurysm, the structure comprises a spheroidal wire basket and the structure comprises a spheroidal foam body.

In an additional aspect, an aneurysm insert is adapted for intravascular delivery to the interior of an aneurysm. The insert comprises a structure that in a rest state intrinsically defines an exterior spheroidal surface. In addition, the structure is compressible to enable passage through a neck of the aneurysm, the structure comprises a spheroidal wire basket and the structure comprises a spheroidal foam body, where the foam body has an internal void.

In another aspect, the insert, as previously described, is adapted to permit the exterior spheroidal surface to adapt to deviations from sphericity in the interior surface of the aneurysm.

In another aspect, the insert, as previously described, further comprises a patch that is adapted substantially to seal the neck of the aneurysm. Thus the structure serves in use to anchor the patch.

In another aspect, the insert, as previously described, further comprises a patch that is adapted substantially to seal the neck of the aneurysm. Thus the structure serves in use to anchor the patch. Furthermore, the structure defines a neck that is contiguous with the spheroidal surface and the patch is separated from the spheroidal surface by the neck.

In an additional aspect, the structure, as previously described, defines a neck contiguous with said spheroidal surface.

In another aspect, the structure, as previously described, has a bulk density that is less than blood density, where the bulk density is the mass of the structure divided by the volume enclosed by the spheroidal surface.

In a further aspect, the structure, as previously described, is porous.

In an additional aspect, the structure, as previously described, is adapted to move to said rest state without inflation.

In another aspect, the structure, as previously described, is self-expanding.

Further objects, features, and advantages of the present invention will become apparent from consideration of the following description and the appended claims when taken in connection with the accompanying drawings.

### BRIEF DESCRIPTION OF THE DRAWINGS

The invention can be better understood with reference to the following drawings and description. The components in the figures are not necessarily to scale, emphasis instead being placed upon illustrating the principles of the invention.
Fig. 1A illustrates a longitudinal cross-sectional view of an aneurysm basket.
Fig. 1B illustrates a three-dimensional view of a distal end of an aneurysm basket.
Fig. 1C illustrates a cross-sectional enlarged view of a wire comprising a plurality of filaments.
Fig. 1D illustrates a longitudinal cross-sectional view of an unwoven aneurysm basket.
Fig. 2A illustrates a longitudinal cross-sectional view of a compressed aneurysm basket 200a, showing the compressed aneurysm basket 200a mounted inside a microcatheter.
Fig. 2B illustrates a longitudinal cross-sectional view of an expanded aneurysm basket, showing the expanded aneurysm basket external to a microcatheter.
Fig. 3A illustrates a longitudinal cross-sectional view of an aneurysm and a parent artery, showing an aneurysm basket deployed within the aneurysm.
Fig. 3B illustrates a three-dimensional view of a proximal basket end.
Fig. 4 illustrates a longitudinal cross-sectional view of an aneurysm basket or anchor member with an attachment located on a basket distal end.
Fig. 5A illustrates a longitudinal cross-sectional view of an Ivalon aneurysm insert.
Fig. 5B illustrates a longitudinal cross-sectional view of an aneurysm insert that is interspersed with a plurality of wires.
Fig. 5C illustrates a longitudinal cross-sectional view of an aneurysm insert, where a body of the aneurysm insert includes an interior region and a void or open space.
Fig. 6 illustrates a longitudinal cross-sectional view of an aneurysm insert, where the insert has an expanded conformation, a compressed conformation and a deployed conformation.
Fig. 7 illustrates a longitudinal cross-sectional view of compressed aneurysm insert that is loaded in a microcatheter and an expanded aneurysm insert.
Fig. 8 is a side environmental view of a sealing device disposed in an aneurysm of a body vessel in accordance with one embodiment of the present invention.
Fig. 9 is an elevated view of the sealing device of Figure 8.
Fig. 10 is an end view of a base of the sealing device in accordance with one embodiment of the present invention.
Fig. 11A is an exploded view of a sealing apparatus for the sealing device in accordance with one embodiment of the present invention.
Fig. 11B is a side view of the aneurysm sealing apparatus in Figure 11A.
Fig. 11C is a side cross-sectional view of the sealing apparatus of Figure 11B.
Fig. 12 is a flow chart depicting one method of sealing a dilatation area of a body vessel caused by an aneurysm.
Fig. 13 is a side cross-sectional view of a sealing apparatus in accordance with another embodiment of the present invention.
Fig. 14 is a side cross-sectional view of a sealing apparatus in accordance with yet another embodiment of the present invention.

### DETAILED DESCRIPTION OF THE DRAWINGS

A device and/or method for the treatment of aneurysms is disclosed herein. This approach involves the occlusion of an aneurysm, such as an intracranial aneurysm, by intravascular insertion of an anchoring device into the aneurysm. The anchoring device may serve to secure a patch in the neck of the aneurysm, where the patch may act to inhibit blood flow from the blood vessel from entering the aneurysm. The patch may serve as a covering material and be derived from a variety of materials, including a collagenous biomaterial and biocompatible polymers. The collagenous biomaterial may serve as a scaffold or matrix for the replacement and repair of the damaged arterial tissue. Thus, unlike previous intravascular aneurysm techniques, the device disclosed herein may achieve occlusion of the aneurysm by stimulating regrowth or repair of the blood vessel wall.

In addition, described examples generally provide a sealing device, sealing apparatus, and methods for sealing a dilatation area of a body vessel formed by an aneurysm. These solve the concerns of current aneurysm treatments, such as the relatively high risks and cost of surgery.

The approach disclosed herein may also reduce the occurrence of mass effect, which can occur with other methods of treatment. In addition, the use of a collagenous biomaterial may lead to a more permanent treatment, which may lower the risk of reformation.

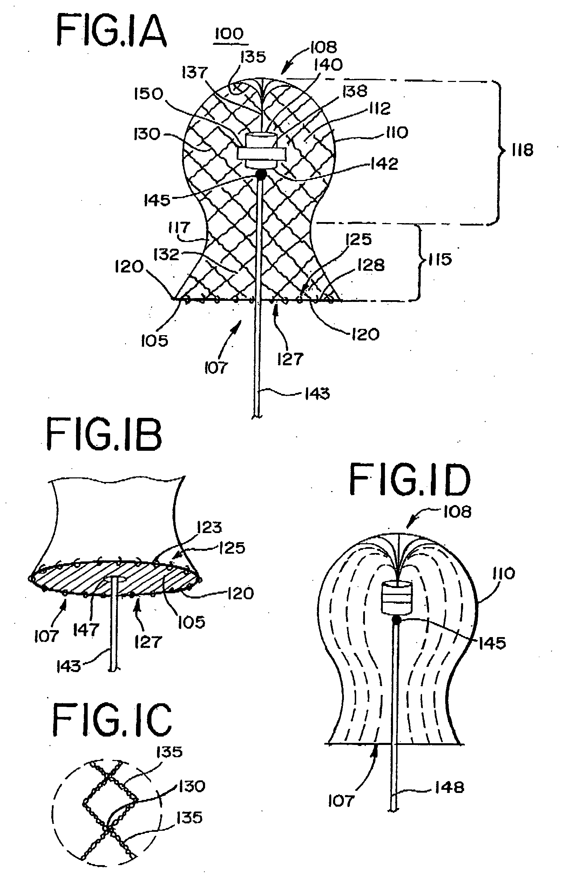

Fig. 1A illustrates a longitudinal cross-sectional view of an aneurysm basket or anchor member 100 with a patch 105, where the aneurysm basket includes a woven basket wall 110. Fig. 1B illustrates a three-dimensional view of a proximal end 107 of the aneurysm basket 100 showing the patch 105. Fig. 1C illustrates a cross-sectional enlarged view a wire 130 comprising a plurality of filaments 135. Fig. 1D illustrates a longitudinal cross-sectional view of an unwoven aneurysm basket 100.

The basket 100 may extend between a proximal basket end 107 and the distal basket end 108, where these may also be referred to as a proximal anchor end 107 and a distal anchor end 108, respectively. The basket wall 110 may be woven (Fig. 1A) or unwoven (Fig. 1D) and may enclose a cavity 112. Where the basket wall 110 is woven (Fig. 1A), and in a further example, the basket 100 may also be described as comprising two mutually orthogonal wire paths. A basket neck region 115 may extend between the proximal basket end 107 and a distal neck end 117. The basket 100 may have a spherical region 118, which extends from the distal neck end 117 to the distal basket end 108. The proximal basket end 107 may include a ring 120. In one example, the ring 120 may be derived from a separate component that is attached to the aneurysm basket 100. In another example, the ring 120 may be formed from the basket 100. When the ring 120 is derived from a separate component, it may be manufactured using a variety of materials, including polymeric materials, shape memory alloys or stainless steel. Shape memory alloys may include a variety of materials such as nitinol (NiTi, nickel-titanium), CuZnAl, CuAINi or similar materials, as are well known in the art. In one example, the shape memory alloy may be nitinol.

In a preferred example, the bulk density of the basket 100 is less than that of blood. The bulk density of the basket 100 is calculated by dividing the mass of the basket 100 by the volume enclosed by the spheroidally shaped basket 100.

A patch 105 may be circumscribed by and attached to the ring 120, where the patch 105 includes a covering of fabric or other flexible material. In one example, the patch 105 may comprise a biocompatible polymeric material, of which there are a wide variety including: polyesters, such as poly(ethylene terephthalate), polylactide, polyglycolide and copolymers thereof; fluorinated polymers, such as polytetrafluoroethylene (PTFE), expanded PTFE and poly(vinylidene fluoride); polysiloxanes, including polydimethyl siloxane; and polyurethanes to name a few.

One example of a polyurethane is THORALON® (THORATEC, Pleasanton, CA), as described in U.S. Pat. Application Publication No. 2002/0065552 A1 and U.S. Pat. No. 4,675,361. According to these patents, THORALON® is a polyurethane base polymer (referred to as BPS-215) blended with a siloxane containing surface modifying additive (referred to as SMA-300). Base polymers containing urea linkages can also be used. The concentration of the surface modifying additive may be in the range of 0.5% to 5% by weight of the base polymer.

The SMA-300 component (THORATEC) is a polyurethane comprising polydimethylsiloxane as a soft segment and the reaction product of diphenylmethane diisocyanate (MDI) and 1,4-butanediol as a hard segment. A process for synthesizing SMA-300 is described, for example, in U.S. Pat. Nos. 4,861,830 and 4,675,361.

The BPS-215 component (THORATEC) is a segmented polyetherurethane urea containing a soft segment and a hard segment. The soft segment is made of polytetramethylene oxide (PTMO), and the hard segment is made from the reaction of 4,4'-diphenylmethane diisocyanate (MDI) and ethylene diamine (ED).

THORALON® can be manipulated to provide either porous or non-porous THORALON®. Non-porous THORALON® can be formed by mixing the polyetherurethane urea (BPS-215) and the surface modifying additive (SMA-300) in a solvent, such as dimethyl formamide (DMF), tetrahydrofuran (THF), dimethyacetamide (DMAC), dimethyl sulfoxide (DMSO). The composition can contain from about 5 wt% to about 40 wt% polymer, and different levels of polymer within the range can be used to fine tune the viscosity needed for a given process. The composition can contain less than 5 wt% polymer for some spray application embodiments. The entire composition can be cast as a sheet, or coated onto an article such as a mandrel or a mold. In one example, the composition can be dried to remove the solvent.

THORALON® has been used in certain vascular applications and is characterized by thromboresistance, high tensile strength, low water absorption, low critical surface tension, and good flex life. THORALON® is believed to be biostable and to be useful in vivo in long term blood contacting applications requiring biostability and leak resistance. Because of its flexibility, THORALON® is useful in larger vessels, such as the abdominal aorta, where elasticity and compliance is beneficial.

A variety of other polyurethanes/polycarbamates and urea linkages (hereinafter "-C(O)N or CON type polymers") may also be employed. These include CON type polymers that preferably include a soft segment and a hard segment. The segments can be combined as copolymers or as blends. For example, CON type polymers with soft segments such as PTMO, polyethylene oxide, polypropylene oxide, polycarbonate, polyolefin, polysiloxane (i.e. polydimethylsiloxane), and other polyether soft segments made from higher homologous series of diols may be used. Mixtures of any of the soft segments may also be used. The soft segments also may have either alcohol end groups or amine end groups. The molecular weight of the soft segments may vary from about 500 to about 5,000 g/mole.

Preferably, the hard segment is formed from a diisocyanate and diamine. The diisocyanate may be represented by the formula OCN-R-NCO, where -R- may be aliphatic, aromatic, cycloaliphatic or a mixture of aliphatic and aromatic moieties. Examples of diisocyanates include MDI, tetramethylene diisocyanate, hexamethylene diisocyanate, trimethyhexamethylene diisocyanate, tetramethylxylylene diisocyanate, 4,4'-dicyclohexylmethane diisocyanate, dimer acid diisocyanate, isophorone diisocyanate, metaxylene diisocyanate, diethylbenzene diisocyanate, decamethylene 1,10 diisocyanate, cyclohexylene 1,2-diisocyanate, 2,4-toluene diisocyanate, 2,6-toluene diisocyanate, xylene diisocyanate, m-phenylene diisocyanate, hexahydrotolylene diisocyanate (and isomers), naphthylene-1,5-diisocyanate, 1-methoxyphenyl 2,4-diisocyanate, 4,4'-biphenylene diisocyanate, 3,3'-dimethoxy-4,4'-biphenyl diisocyanate and mixtures thereof.

The diamine used as a component of the hard segment includes aliphatic amines, aromatic amines and amines contaning both aliphatic and aromatic moieties. For example, diamines include ethylene diamine, propane diamines, butanediamines, hexanediamines, pentane diamines, heptane diamines, octane diamines, m-xylylene diamine, 1,4-cyclohexane diamine, 2-methypentamethylene diamine, 4,4'-methylene dianiline, and mixtures thereof. The amines may also contain oxygen and/or halogen atoms in their structures.

Other applicable polyurethanes include those using a polyol as a component of the hard segment. Polyols may be aliphatic, aromatic, cycloaliphatic or may contain a mixture of aliphatic and aromatic moieties. For example, the polyol may be ethylene glycol, diethylene glycol, triethylene glycol, 1,4-butanediol, 1,6-hexanediol, 1,8-octanediol, propylene glycols, 2,3-butylene glycol, dipropylene glycol, dibutylene glycol, glycerol, or mixtures thereof.

CON type polymers modified with cationic, anionic and aliphatic side chains may also be used. See, for example, U.S. Pat. No. 5,017,664.

Other CON type polymers include: segmented polyurethanes, such as BIOSPAN®; polycarbonate urethanes, such as BIONATE®; and polyetherurethanes, such as ELASTHANE®; (all available from POLYMER TECHNOLOGY GROUP, Berkeley, CA).

Other CON type polymers can include polyurethanes having siloxane segments, also referred to as a siloxane-polyurethane. Examples of polyurethanes containing siloxane segments include polyether siloxane-polyurethanes, polycarbonate siloxane-polyurethanes, and siloxane-polyurethane ureas. Specifically, examples of siloxane-polyurethane include polymers such as ELAST-EON 2® and ELAST-EON 3® (AORTECH BIOMATERIALS, Victoria, Australia); polytetramethyleneoxide (PTMO) and polydimethylsiloxane (PDMS) polyether-based aromatic siloxane-polyurethanes such as PURSIL-10®, -20, and -40 TSPU; PTMO and PDMS polyether-based aliphatic siloxane-polyurethanes such as PURSIL AL-5® and AL-10 TSPU; aliphatic, hydroxy-terminated polycarbonate and PDMS polycarbonate-based siloxane-polyurethanes such as CARBOSIL-10®, -20, and -40 TSPU (all available from POLYMER TECHNOLOGY GROUP). The PURSIL®, PURSIL-AL®, and CARBOSIL® polymers are thermoplastic elastomer urethane copolymers containing siloxane in the soft segment, and the percent siloxane in the copolymer is referred to in the grade name. For example, PURSIL-10 contains 10% siloxane. These polymers are synthesized through a multi-step bulk synthesis in which PDMS is incorporated into the polymer soft segment with PTMO (PURSIL®) or an aliphatic hydroxy-terminated polycarbonate (CARBOSIL®). The hard segment consists of the reaction product of an aromatic diisocyanate, MDI, with a low molecular weight glycol chain extender. In the case of PURSIL-AL® the hard segment is synthesized from an aliphatic diisocyanate. The polymer chains are then terminated with a siloxane or other surface modifying end group. Siloxane-polyurethanes typically have a relatively low glass transition temperature, which provides for polymeric materials having increased flexibility relative to many conventional materials. In addition, the siloxane-polyurethane can exhibit high hydrolytic and oxidative stability, including improved resistance to environmental stress cracking. Examples of siloxane-polyurethanes are disclosed in U.S. Pat. Application Publication No. 2002/0187288 A1.

In addition, any of these CON type polymers may be end-capped with surface active end groups, such as, for example, polydimethylsiloxane, fluoropolymers, polyolefin, polyethylene oxide, or other suitable groups. See, for example the surface active end groups disclosed in U.S. Pat. No. 5,589,563.

In another example, the patch 105 may comprise a collagenous biomaterial, such as small intestine submucosa (SIS). In a further example, the patch 105 may consist of a porous biocompatible polymer in which a collagenous biomaterial has been dispersed, as is disclosed in U.S. Provisional Application Serial No. 60/558,794 filed March 31, 2004 and U.S. Provisional Application Serial No. 60/558,667 filed March 31, 2004 (the benefit of which is claimed in U.S. Patent Application Publication No. 2005/0220848). The patch 105 may be attached to the ring 120 using a variety of attachment techniques. These attachment techniques may include sutures, adhesive, heat sealing, "weaving" together, crosslinking, or other known methods of attachment. In one example, the patch 105 is attached to the ring 120 by a plurality of sutures 123. The patch 105 may include a patch distal side 125 and a patch proximal side 127.

Connective tissue or collagenous biomaterials may be derived from a variety of sources, including vertebrate submucosa tissue. This material is a complex structural entity surrounding and supporting cells that are found within mammalian tissues, which may include naturally associated extracellular matrix proteins, glycoproteins and other factors. Furthermore, this material comprises structural proteins (e.g., collagen and elastin), specialized protein (e.g., fibrillin, fibronectin and laminin), and proteoglycans, a protein core to which are attached long chains of repeating disaccharide units referred to as glycosaminoglycans. This material has been employed successfully in a variety of procedures, including vascular grafts, urinary bladder and hernia repair, replacement and repair of tendons and ligaments, and dermal grafts. In fact, when collagenous biomaterials, such as vertebrate submucosa, which includes small intestine submucosa (SIS), is used as a tissue graft it appears to serve both as a matrix for the regrowth of the replaced tissues and to induce growth of endogenous tissues. Common events to this remodeling process include: widespread and very rapid neovascularization, proliferation of granulation mesenchymal cells, biodegradation/resorption of implanted intestinal submucosal tissue material, and lack of immune rejection.

Submucosal tissue can be obtained from various sources, including intestinal tissue harvested from animals raised for meat production, including, for example, pigs, cattle and sheep or other warm-blooded vertebrates. This tissue can be used in either its natural configuration or in a comminuted or partially digested fluidized form. Vertebrate submucosal tissue is a plentiful by-product of commercial meat production operations and is thus a low cost cell growth substrate. When employed as a tissue graft composition, SIS provides excellent mechanical properties, including high compliance, a high burst pressure point, and an effective porosity index.

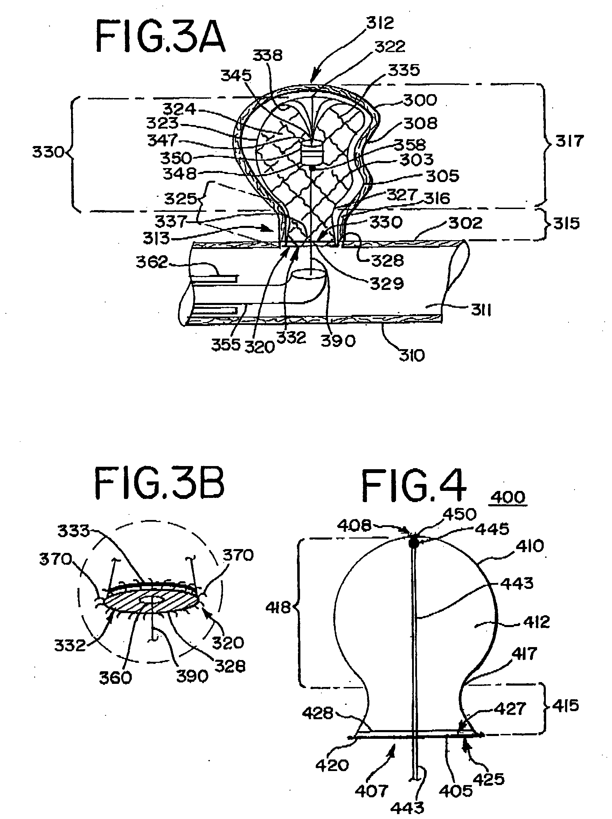

The basket 100 may be deployed in a variety of aneurysms, such as for example intracranial aneurysms. When the basket 100 is deployed in an intracranial aneurysm, it may occupy the aneurysm as shown in Fig. 3. Thus, with the patch 105 located in substantial alignment with the blood vessel wall, the collagenous biomaterial may stimulate re-growth of the arterial wall, while being anchored in place by the basket. This regrowth may occur such that the arterial wall grows from the parent artery into the patch 105, eventually converting the collagenous biomaterial patch 105 into arterial wall and providing a continuous arterial wall. Replacement of the collagenous biomaterial by the arterial wall may provide a more permanent seal or occlusion of the aneurysm.

The thickness of the patch 105 may vary, depending on an assortment of factors. For example, the selection of a thickness may be based on the purpose for which the basket 100 will be used, the location where the basket 100 will be employed, the blood pressure present at the deployment location and/or the size and/or shape of the aneurysm. The thickness of the patch 105 may be regulated in a number of ways. For example, one way to increase the thickness of the patch 105 is to use multiple layers of the collagenous biomaterial, which may or may not be laminated together. When the patch 105 is a collagenous biomaterial, it may include a plurality of layers, such that the thickness of the patch 105 may be increased by increasing the number of layers.

The size of the patch 105 may be modified in conjunction with the size of the ring 120. For example, the size of the patch 105 may be chosen such that it substantially occupies the area enclosed by the ring 120. The size of the ring 120 may vary depending on a number of factors, including the size and/or shape of the aneurysm neck (See Fig. 3A). The size of the ring 120 may be selected to provide a snug fit between the aneurysm neck and the proximal basket end 108. Providing a snug fit between the proximal basket end 107 and the aneurysm may be an important factor in inhibiting blood flow into the aneurysm. Inhibition of blood flow into the aneurysm may slow or hinder the growth of the aneurysm and the risk of aneurysm rupture.

The patch 105 may be reinforced with a reinforcement material 128, where the reinforcement material 128 may serve to provide structural support. The reinforcement material 128 may be designed such that it is capable of being compressed when the basket 100 is contained within a microcatheter. In one example, the reinforcement material 128 may be applied to the surface of the patch 105, either on the patch distal side 125 or the patch proximal side 127 or both. In another example, the reinforcement material may be situated within the patch 105. The reinforcement material 128 may include a variety of materials, including shape memory alloys, stainless steel, and polymeric materials. In one example, the reinforcement material 128 may include nitinol. In another example, the reinforcement material 128 may be a woven or unwoven. Additionally, the reinforcement material 128 may be attached to the ring 120.

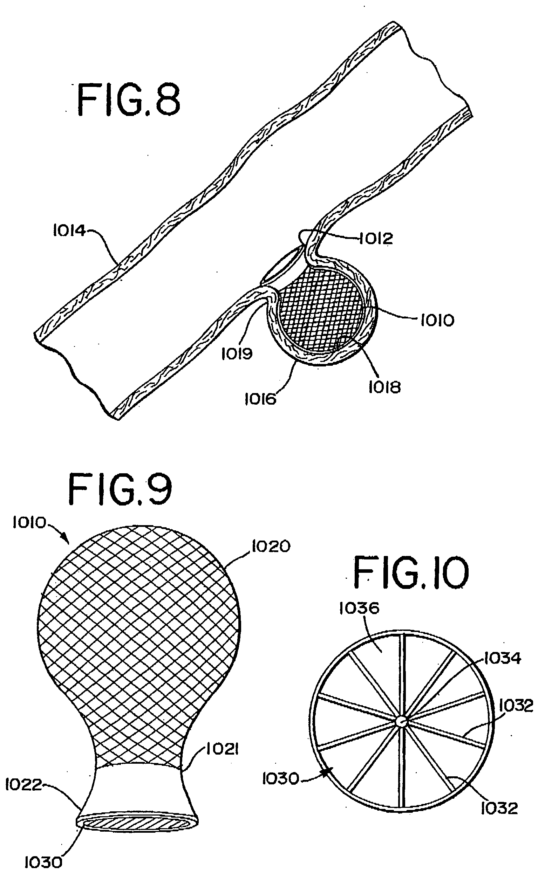

The patch 105 may extend beyond the ring 120 and may overlap or the neck region 115 (for example see the neck portion 1021 in Fig. 9). In this manner, the patch 105 may have an increased interaction with the arterial wall and the neck of the aneurysm, which may be particularly beneficial when the patch 105 includes collagenous biomaterial. Furthermore, this may provide an enhanced seal between the ring 120, and the neck region 115, the neck of the aneurysm and the arterial wall. It is important to note that forming a tight seal between the proximal portions of the aneurysm basket 100 (such as the ring 120 and the aneurysm neck 115) may provide a better treatment result.

The basket 100 may include a plurality of wires 130, where wires 130 may form the basket wall 110. The wires 130 may extend between a proximal wire end 132 and a distal wire end 137. In one example, the proximal wire ends 132 may be woven together to form the ring 120. In another example, the proximal wire ends 132 may be attached to the ring 120. The wires 130 may be woven to provide the basket wall 110 as shown in Fig. 1A. In another example, the wire 130 may be unwoven to provide the basket wall 110, as shown in Fig. 1D. In one example, the basket 100 may have from 4 to 15 wires 130. However, the basket 100 may also have from 4 to 50 wires 130. The basket 100 may also have from 4 to 25 wires 130. Other variations are also possible.

Each wire 130 may include a filament 135 or a plurality of filaments 135. In one example, where the wires 130 include a plurality of filaments 135, the plurality of filaments 135 may be twisted or braided to form the wire 130. The filaments 135 may comprise a variety of materials, including polymer materials, shape memory alloys or stainless steel. In one example, the filaments 135 may be nitinol. In one example, the wires 130 may have a diameter ranging from about 0.003 cm (0.001 in.) to about 0.254 cm (0.100). However, the wires 130 may also have a diameter ranging from about 0.025 cm (0.010 in.) to about 0.229 cm (0.090 in.). Other variations are also possible.

The distal wire ends 137 may extend from the distal basket end 108 into the cavity 112 and may be joined together within the cavity 112. For example, the distal wire ends 137 may be fastened together within a cannula 138, where the cannula 138 may also be located in the cavity 112. The cannula 138 may extend between a distal cannula end 140 and a proximal cannula end 142 and may be made from a variety of materials including polymer materials, shape memory alloy, or stainless steel.

Whether the basket 100 is unwoven, as illustrated in Fig. 1D, or woven, as illustrated in Fig. 1A, a variety of delivery methods may be employed. For example, the basket 100 may be delivered via a pusher wire 143, where the pusher wire 143 can be formed from a variety of materials, such as stainless steel. The pusher wire 143 may be connected to the basket 100 by way of an attachment 145. The attachment 145 may be integral with the cannula 138 or it may be attached to the cannula 138. The combination of the attachment 145 and the cannula 138 may be referred to as a releasable member. The attachment 145 may be manipulated such that the wire pusher 143 may undergo selective detachment from the cannula end 138. Furthermore, this manipulation may be initiated externally to the patient. Selective detachment of the attachment 145 may occur via a variety of mechanisms, including, for example, a mechanical mechanism or an electrolytic mechanism. In one example, the pusher wire 143 may be attached to the cannula 138, where the attachment 145 is formed from a segment of solder. Detachment may be externally initiated by application of an electric current to the pusher wire 143, where the electric current may result in dissolution of the attachment 145, for example the solder. In another example, the basket 100 may be delivered using cup biopsy forceps (not shown). This delivery technique may function by enclosing the proximal basket end 107 within the closed cup biopsy forceps, while the remainder of the basket 100 remains distal to and on the exterior of the closed cup biopsy forceps. The basket 100 may then be released by opening the cup biopsy forceps. Utilization of the cup biopsy forceps for delivery may make the attachment 145 and the pusher wire 143 unnecessary components.

To provide an access point for the pusher wire 143 the patch 105 may have a pusher wire access point 147, which may be located near the center of the patch 105 and may extend between the patch proximal side 125 and the patch distal side 127 of the patch 105. In one example, the access point 147 may be a slit or an aperture that allows the pusher wire 143 to pass through the patch 105. The access point 147 may be designed to allow the pusher wire 143 to pass through it, while also minimizing the amount of blood that can flow into the aneurysm.

The shape and/or size of the basket 100 and its individual components may be an important factor in ensuring that the basket 100 securely anchors the patch 105 in the neck of the aneurysm. For example, the basket 100 may serve to anchor the patch 105 by engaging the inner surface of the aneurysm. The shape of the spherical region 118 may be generally spherical. However, the shape and/or size may vary based on the shape and/or size of the aneurysm. In one example, the aneurysm basket 100 may be shaped, prior to deployment. In another example, the basket 100 may be flexible such that the shape of the basket will substantially conform to the shape of the aneurysm upon deployment of the basket 100. The ability of the basket 100 to conform to the shape and/or size of the aneurysm and the ability of the basket 100 to exert some amount of radial force on the aneurysm may ensure that the basket 100 is securely anchored within the aneurysm.

In one example, the length, shape and/or size of the neck region 115 may vary, depending on the length, shape and/or size of the aneurysm to be treated. In another example, the shape of the spherical region 118 may be generally spherical. However, the shape and/or size of the spherical region 118 may vary depending on the shape and/or size of the aneurysm to be treated.

The aneurysm basket 100, disclosed herein, may serve to anchor the patch 105 in the neck of the aneurysm. However, unlike microcoils, the basket 100 does not fill the aneurysm with a solid or semisolid mass. Instead, the basket 100 may be hollow, relatively light, and pliable or flexible. Consequently, the basket 100 may function to occlude the aneurysm, while also reducing the occurrence of mass effect.

Movement of the basket 100 may be tracked by providing a radiopaque basket marker 150. The basket marker 150 may be located in a number of areas on the basket 100. For example, the basket marker 150 may be located on the cannula 138.

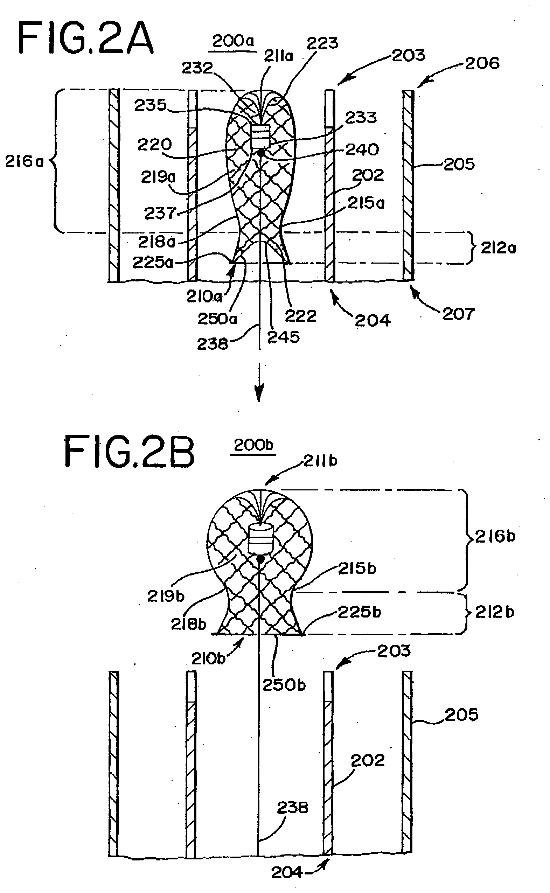

Fig. 2A illustrates a longitudinal view of a compressed aneurysm basket or compressed anchor member 200a, showing the compressed aneurysm basket 200a mounted inside a microcatheter 202. The aneurysm basket 200a can be converted to an expanded aneurysm basket upon expulsion from the microcatheter 202, as shown in Fig. 2B. Thus the basket 200a is in a collapsed state, while the basket 200b is in an expanded state. The microcatheter 202, which may have a microcatheter distal end 203 and a microcatheter proximal end 204, may be employed in conjunction with an angiographic catheter 205. The angiographic catheter 205 may include an angiographic distal end 206, an angiographic proximal end 207. The angiographic catheter 205 may serve as a guide for the microcatheter 202. For example, the angiographic catheter distal end 206 may be directed to a generally desired location, such as the general location of an aneurysm (not shown), while the angiographic proximal end 207 remains located externally to a patient. Next, the microcatheter 202 may be fed into the angiographic proximal end 207 and through the angiographic catheter 205. When the microcatheter distal end 203 reaches the angiographic distal end 206, the microcatheter distal end 203 may be extended beyond the angiographic distal end 206, such that the microcatheter distal end 203 arrives at the desired location in the patient. The microcatheter distal end 203 may then be guided into the aneurysm for deployment of the compressed basket 200a.

The compressed basket 200a may comprise a compressed proximal basket end 210a and a compressed distal basket end 211a, where these may also be referred to as a compressed proximal anchor end 210a and a compressed distal anchor end 211a, respectively. The compressed basket 200a may also have a compressed neck region 212a, extending between the compressed proximal basket end 210a and a compressed distal neck end 215a. The compressed basket 200a may have a compressed spherical region 216a, which extends from the compressed distal neck end 215a to the distal basket end 211a. In addition, the compressed basket 200a may include a compressed basket wall 218a, extending from the compressed proximal basket end 210a to the compressed distal basket end 211a. Furthermore, the compressed basket 200a may include a compressed cavity 219a, where the compressed cavity 219a is defined by the compressed basket wall 218a.

The compressed basket 200a may comprise a plurality of wires 220, where the wires 220 may form the compressed basket wall 218a. The wires 220 may each have a proximal wire end 222 and a distal wire end 223. The proximal wire ends 222 may be woven together to form a compressed ring 225a, where the compressed ring 225a may form the compressed proximal basket end 210a. In one example, the wires 220 may be woven to provide the compressed basket wall 218a in a woven configuration, as shown in Fig. 1A. In another example, the wires 220 may be unwoven as shown in Fig. 1D. In one example, the compressed basket 200a may have from 4 to 15 wires 220. The compressed basket 200a may also have from 4 to 50 wires 220. However, the compressed basket 200a may have from 4 to 25 wires 220.

Each wire 220 may be formed from a filament or a plurality of filaments, such as filaments 135 as shown in Fig. 1C. When each wire 220 includes a plurality of filaments, the plurality of filaments may be twisted or braided to form each of the wires 220. The plurality of filaments may be constructed from a variety of materials, including polymeric materials, shape memory alloys, or stainless steel. In a preferred example, the plurality of filaments comprise a shape memory alloy, such as nitinol (NiTi, nickel-titanium), CuZnAl, and CuAINi or a similar material, as is well known in the art. In a more preferred example, the plurality of wires 220 are made of nitinol. In another example, the plurality of wires 220 may have a diameter of about 0.003 cm (0.001). to about 0.254 cm (0.100 in.). In another example, the plurality of wires 220 may have a diameter of about 0.025 cm (0.010 in.) to 0.229 cm (0.090 in). Other variations are also possible.

The distal wire ends 223 may extend from compressed distal basket end 211a into the compressed cavity 219a. The distal wire ends 223 may be joined together within the compressed cavity 219a to form a collection of distal wire ends 232. In one example, the collection of distal wire ends 232 may be fastened together within a cannula 233, where the cannula 233 is also located within the compressed cavity 219a. The cannula 233 may have a distal cannula end 235, a proximal cannula end 237, and may be constructed from a variety of materials, including polymeric materials, shape memory alloys, or stainless steel.

The compressed basket 200a may be connected to a pusher wire 238, where the pusher wire 238 is connected by way of an attachment 240. The attachment 240 may be located on or may be integral with the cannula 233 and may be manipulated such that the wire pusher 238 can undergo selective detachment from the cannula end 233. When the attachment 240 is integral with the cannula 233, this combination may be referred to as a releasable member. This manipulation may be initiated externally to the patient and may result in detachment of the basket 200 from the microcatheter 205. The attachment 240 may function via a variety of mechanisms, including for example a mechanical mechanism or an electrolytic mechanism.

Movement of the compressed basket 200a may be tracked by providing a radiopaque marker 243. In one example, the marker 243 may be located on the cannula 233.

The compressed basket 200a may include a collapsed patch 250a, where the collapsed patch 250a may be attached to the collapsed ring 225a. The collapsed patch 250a may be attached to the collapsed ring 225a by a variety of techniques, such as crimping or by sutures. In one example, the collapsed patch 250a may be tucked into the compressed cavity 219a.

To provide an access point for the pusher wire 238 the collapsed patch 250a may have a pusher wire access point 245, where the access point 245 is located near the center of the collapsed patch 250a. In one example, the access point 245 may be a slit or an aperture that allows the pusher wire 238 to pass through the collapsed patch 250a to connect to the proximal cannula end 237.

Fig. 2B illustrates a longitudinal cross-sectional view of the expanded aneurysm basket 200b, showing the expanded aneurysm basket 200b external to the microcatheter 202. The compressed basket 200a may possess a radial force such that the compressed basket 200a will expand radially when expelled from the microcatheter 202. Thus, once the microcatheter 202 is guided into the aneurysm, the compressed basket 200a may be expelled by extending the pusher wire 238 to provide the expanded basket 200b, as well as an expanded proximal basket end 210b, an expanded distal basket end 211b, an expanded neck region 212b, an expanded distal neck end 215b, an expanded spherical region 216b, an expanded basket wall 218b, an expanded cavity 219b, an expanded ring 225b, and an expanded patch 250b. The expanded proximal basket end 210b and the expanded distal basket end 211b may also be referred to as the expanded proximal anchor end 210b and the expanded distal anchor end 211b, respectively. The expanded basket 200b may occupy the larger space of the aneurysm. In some cases it may be desirable for the expanded basket 200b to retain some amount of radial force, even after being deployed into the aneurysm, since this may contribute to securely anchoring the patch 250b.

Fig. 3A illustrates a longitudinal cross-sectional view of an aneurysm 300 and a parent artery 302, showing the aneurysm basket 303 deployed within the aneurysm 300. Fig. 3B illustrates a three-dimensional view of a proximal basket end 320.

The aneurysm 300 may include an aneurysm wall 305 and one or more aneurysm irregularity(ies) 308. The aneurysm 300 is a blood-filled dilation of the parent artery 302, where the parent artery 302 includes an arterial wall 310 that defines an arterial lumen 311. The aneurysm 300 may be defined by an aneurysm distal end 312 and an aneurysm proximal end 313, where an aneurysm neck region 315 extends from the aneurysm proximal end 313 to a distal aneurysm neck end 316. An aneurysm body 317 may extend from the aneurysm neck region 315 to the aneurysm distal end 312.

The basket 303 may extend between a proximal basket end 320 and a distal basket end 322, where these may also be referred to as a proximal anchor end 320 and a distal anchor end 322, respectively. The basket 303 may include a basket wall 323, where the basket wall 323 may enclose a cavity 324. A basket neck region 325 may extend between the proximal basket end 320 and a distal neck end 327. The basket 303 may have a spherical region 330, which extends from the distal neck end 327 to the distal basket end 322. The proximal basket end 320 may include a ring 328. In one example, the ring 328 may be derived from a separate component that is attached to the aneurysm basket 303 or the ring 328 may be formed from the basket 303. When the ring 328 is derived from a separate component, it may be manufactured using a variety of materials, including, polymeric materials, shape memory alloys, or stainless steel. In one example, the shape memory alloy may be nitinol.

A patch 329 may be circumscribed by, and attached to, the ring 328, where the patch 329 includes a covering of fabric or other flexible material. The patch 329 may comprise a variety of materials as are described for patch 105 in Fig. 1A. The patch 329 may be attached to the ring 328 using a variety of attachment techniques, which may include sutures, adhesive, heat sealing, "weaving" together, crosslinking, or other known methods of attachment. In one example, the patch 329 may be attached to the ring 329 by a plurality of sutures, such as sutures 123 shown in Fig. 1B. The patch 329 may include a patch distal side 330 and a patch proximal side 332. The patch 329 may serve as a barrier to blood flow between the aneurysm 300 and the parent artery 302. When the patch 329 includes the collagenous biomaterial, such as small intestine submucosa, the arterial wall of the parent artery 302 may replace the collgenous biomaterial and permanently seal the aneurysm 300

The thickness of the patch 329 may be altered, depending on an assortment of factors. For example, the selection of a thickness may be based on the purpose for which the basket 303 will be used, the location where the basket 303 will be employed, and the blood pressure present at the deployment location. The thickness of the patch 329 may be regulated in a number of ways. For example, when the patch 329 is collagenous biomaterial, the thickness of the patch 329 may be increased by using multiple layers.

The size of the patch 329 may be modified in conjunction with the size of the ring 328. For example, the size of the patch 329 may be chosen such that it substantially occupies the area circumscribed by the ring 328. The size of the ring 328 may vary depending on a number of factors, including the size of the aneurysm neck 315. For example, the size of the ring 328 may be selected to provide a snug fit between the aneurysm neck 315 and the proximal basket end 320. A snug fit between the proximal basket end 320 and the aneurysm neck 315 may minimize the blood flow from the parent artery 302 into the aneurysm 300. A reduction in blood flow from the parent artery 302 into the aneurysm 300 may reduce the blood pressure within the aneurysm 300 and may concomitantly inhibit further growth of the aneurysm 300. Additionally, reduced blood flow into the aneurysm 300 may encourage formation of a thrombosis in the aneurysm 300, which may also discourage further growth of the aneurysm 300. Placement of the proximal basket end 320 and the ring 328 may also be an important factor in discouraging further growth of the aneurysm 300 and for encouraging formation of a thrombosis. For example, in some cases it may be beneficial to locate the proximal basket end 320 near or in alignment with the aneurysm proximal end 313.

The patch 329 may be reinforced with a reinforcement material 333, where the reinforcement material 333 may serve to provide structural support. For example, the reinforcement material 333 may ensure that the patch 329 is not deformed and thus forced back into the cavity 324 by the blood pressure present in the parent artery 302. Additionally, the reinforcement material 333 may ensure that the patch 329 is not ripped or damaged by the forces exerted against it by the blood pressure present in the parent artery 302. In one example, the reinforcement material 333 may be applied to the surface of the patch 329, either on the patch distal side or the patch proximal side or both. In another example, the reinforcement material may be situated within the patch 329. The reinforcement material 333 may include a variety of materials, including polymeric materials, shape memory alloys or stainless steel. In one example, the reinforcement material 333 may include nitinol. In one example, the reinforcement material 333 may be a woven wire mesh. In another example, the reinforcement material 333 may be a plurality of reinforcement wires. The reinforcement material 333 may be anchored by attachment to the ring 328.

The basket 303 may include a plurality of wires 335, which may extend between a plurality of proximal wire ends 337 and a plurality of distal wire ends 338. In one example, the proximal wire ends 337 may be woven together to form the ring 328. In another example, the proximal wire ends 337 may be attached to the ring 328. The wires 335 may form the basket wall 323. In one example, the wires 335 may be woven to provide the basket wall 323, as shown in Fig. 1A. In another example, the wires 335 may be unwoven to provide the basket wall 323, as shown in Fig. 1D. In one example, the basket 303 may comprise from 4 to 15 wires 335. In another example, the basket 303 may comprise from 4 to 50 wires 335. In a further example, the basket 303 may comprise from 4 to 25 wires 335.

Each wire 335 may further comprise a filament or a plurality of filaments, such as filaments 135 as shown in Fig. 1C. In one example, where the wires 335 include a plurality of filaments, the plurality of filaments may be twisted or braided to form the wires 335. The filaments may be made from a variety of materials. For example, the filaments 30 may be made from polymeric materials, such as PTFE or polyester. Additionally, the filaments may be made from stainless steel or from shape memory alloys, such as nitinol. In one example, the wires 335 may have a diameter ranging from 0.003 cm (0.001 in.) to 0.254 cm (0.100 in.). In another example, the wires 335 may have a diameter ranging from about 0.025 cm (0.010 in.) to about 0.229 cm (0.090 in). Other variations are also possible.

A plurality distal wire ends 338 may extend from the distal basket end 322 into the cavity 324. The distal wire ends 338 may be joined together within the cavity 324 to form a collection of distal wire ends 345. In one example, the collection of distal wire ends 345 may be fastened together within a cannula 350, where the cannula 350 may also be located in the cavity 324. The cannula 350 may extend between a distal cannula end 347 and a proximal cannula end 348, and may be constructed from a variety of materials, including polymeric materials, shape memory alloys or stainless steel.

The basket 303, as disclosed herein, with its hollow cavity 324 and its wire construction 335, may be relatively light and pliable, in comparison to the coil mass formed by micro-coil thrombosis treatment techniques. Consequently, the basket 303 may function to occlude the aneurysm 300, while also reducing the occurrence of mass effect.

The aneurysm basket 303 may be associated with a microcatheter 355 via a pusher wire 390. The pusher wire 390 may be connected to the basket 303 by way of an attachment 358. In one example, the attachment 358 may be located on the proximal cannula end 348. In another example, the attachment 358 may be integral with the cannula 348. When the attachment 358 is integral with the cannula 348, this combination may be referred to as a releasable member. The attachment 358 may be manipulated such that the wire pusher 390 may undergo selective detachment from the proximal cannula end 348. This manipulation may be initiated externally to the patient and may result in detachment of the basket 303 from the microcatheter 355. Selective detachment of the attachment 358 may occur via a variety of mechanisms, including, for example, a mechanical mechanism or an electrolytic mechanism.

To provide an access point for the pusher wire 352 the patch 329 may have a pusher wire access point 360. The pusher wire access point 360 may be located near the center of the patch 329 and may extend from the patch proximal side 332 to the patch distal side 330 of the patch 329. In one example, the access point 360 may be a slit or an aperture that allows the pusher wire 390 to pass through the patch 329. In one example, the size of the access point 360 may be designed to minimize blood flow from the arterial lumen 311 into the basket cavity 324.

In one example, deployment of the aneurysm basket 303 may begin with insertion of the distal end of an angiographic catheter or wire guide catheter 362 into the femoral artery of a patient using the Seldinger technique. The distal end of the angiographic catheter may then be directed through the arterial system of the patient to the general location of the aneurysm 300, while the proximal end of the angiographic catheter 362 remains external to the patient. Next, the distal end of the microcatheter 355 may be fed into the proximal end of the angiographic catheter 362 and the microcatheter 355 may be advanced through the angiographic catheter 362. As with the angiographic catheter 362, the proximal end of the microcatheter 355 may remain external to the patient. The distal end of the microcatheter 355 may be advanced out of the distal end of the angiographic catheter 362 and into the aneurysm 300. Once the distal end of the microcatheter 355 is located inside the aneurysm 355, the pusher wire 390 may be used to expel the compressed basket (not shown) out of the microcatheter 355 and into the aneurysm 300. As the basket is expelled from the distal end of the microcatheter 355, it may expand to substantially occupy the aneurysm 300. In one example, when the basket 303 is deployed within the aneurysm 300 it may still possess some amount of radial force, such that the basket 300 may fit snugly within the aneurysm 300 and thus anchor the patch 308 in the proximal aneurysm end 313.

In one example, the aneurysm basket 303 may conform to the aneurysm irregularity or irregularities 308. Thus although the shape of the spherical region 330 may be generally spherical. The shape and/or size may vary, based on the shape and/or size of the aneurysm 300 and the shape and/or size of irregularities 308. In one example, the aneurysm basket 303 may be shaped, prior to deployment, such that the aneurysm basket 303 will substantially conform to the aneurysm irregularities 308. In another example, the basket 303 may be flexible such that the shape of the basket will substantially conform to the shape of the aneurysm 300 and/or the aneurysm irregularities 308 upon deployment of the basket 303. The ability of the basket 303 to conform to the shape and/or size of the aneurysm 300 and the ability of the basket 303 to exert some amount of radial force on the aneurysm 300, following deployment, may ensure that the basket 303 is securely anchored within the aneurysm 300. This in turn may be a factor in ensuring that the proximal basket end 320 and the patch 329 are securely anchored substantially within the aneurysm proximal end 313 such that the proximal basket end 320 and the patch 329 are not forced out of the aneurysm proximal end and into the aneurysm body 317 by the forces exerted by the blood flow in the parent artery. Ensuring a secure fit for the proximal basket end 320 and the patch 327 may also help to provide a secure or snug fit between the proximal aneurysm end 313 and the proximal basket end 320.

In one example, the length, shape and/or size of the neck region 325 may vary, depending on the length, shape and/or size of the aneurysm 300 and/or the aneurysm neck region 315. In another example, the shape and/or size of the spherical region 330 may vary depending on the shape and/or size of the aneurysm 300 and the aneurysm body 317.

Additional anchoring of the basket 303 may be achieved by attaching barbs 370 to the surface of the basket 303 and/or to the ring 328 (see Fig. 3B). In one example, the barbs may be located on the proximal basket end 320 to secure the basket 303 within the aneurysm 300 and to reduce possible blood flow from the parent artery, into the aneurysm 300. The barbs 370 may include wires, hooks, or any structure attached to the frame and configured so as to be capable of anchoring the basket 303 within the aneurysm 300. In one example, the barbs 370 may anchor the basket 303 by piercing the tissue of the aneurysm 300.

Fig. 4 illustrates a longitudinal cross-sectional view of an aneurysm basket or anchor member 400 with an attachment 445 located on a distal basket end 408.

The basket 400 may extend between a proximal basket end 407 and a distal basket end 408, where these may also be referred to as a proximal anchor end 407 and a distal anchor end 408, respectively. The basket 400 includes a basket wall 410. The basket wall 410 may enclose a cavity 412. A basket neck region 415 may extend between the proximal basket end 407 and a distal neck end 417. The basket 400 may have a spherical region 418, which extends from the distal neck end 417 to the distal basket end 408. The proximal basket end 407 may include a ring 420. In one example, the ring 420 may be derived from a separate component that is attached to the aneurysm basket 400. In another example, the ring 420 may be formed from the basket 400. When the ring 420 is derived from a separate component, it may be manufactured using a variety of materials, including, polymeric materials, shape memory alloys, or stainless steel. Shape memory alloys may include a variety of materials such as nitinol (NiTi, nickel-titanium), CuZnAl, and CuAINi or similar materials, as are well known in the art. In one example, the shape memory alloy may be nitinol.

A patch 405 may be circumscribed by, and attached to, the ring 420, where the patch 405 may comprise a variety of materials as are described for patch 105 in Fig. 1A. The patch 405 may be attached to the ring 420 using a variety of attachment techniques. These attachment techniques may include sutures, adhesive, heat sealing, "weaving" together, crosslinking, or other known methods of attachment. In one example, the patch 405 may be attached to the ring 420 by a plurality of sutures 423. The patch 405 may include a patch distal side 425 and a patch proximal side 427.

In one example, the patch 405 may be reinforced with a reinforcement material 428 where the reinforcement material 428 may serve to provide structural support. In one example, the reinforcement material 428 may be applied to the surface of the patch 429, either on the patch distal side 425 or the patch proximal side 427 or both. In another example, the reinforcement material may be situated within the patch 405. The reinforcement material 428 may comprise a variety of materials, including shape memory alloys or stainless steel. In a preferred example, the reinforcement material 428 is made of nitinol. In another example, the reinforcement material 428 may be a woven or unwoven. The reinforcement material 428 may be attached to the ring 420.

The basket 400 may include a plurality of wires (see Fig. 1A) that may be woven (see Fig. 1A) or unwoven (see Fig. 1D), however for clarity the wires are not shown in Fig. 4. In addition, although the basket 400 may be woven or unwoven, the basket 400 does not possess distal wire ends, such as the distal wire ends 137 shown in Fig. 1A. As a result, there are no distal wire ends that extend from the distal basket end 408 into the cavity 412 and there is no cannula, such as cannula 138 in Fig. 1A. The construction of the basket 400 may allow it to be collapsed, such that the basket 400 may be loaded into a microcatheter for delivery (See Figs. 2A and 2B).

A variety of delivery methods may be employed. In one example of a method, the aneurysm basket 400 may be delivered via a pusher wire 443, where the pusher wire 443 may be connected to the basket 400 by way of an attachment 445. In this example of a method, the attachment 445 may be located within the cavity 412 and on the basket distal end 408.
However, there is no cannula 140, as shown in Fig. 1A, separating the attachment 445 and the basket distal end 408. The attachment 445 may be manipulated such that the pusher wire 443 may undergo selective detachment at the attachment 445. This manipulation may be initiated externally to the patient. Selective detachment of the attachment 445 may occur via a variety of mechanisms, including for example, a mechanical mechanism or an electrolytic mechanism.

To provide an access point for the pusher wire 443 the patch 405 may have a pusher wire access point (see Fig. 1B, pusher wire access point 147) to allow the pusher wire 443 to pass through the patch 405.

In another example of a method, the basket 400 may be delivered using cup biopsy forceps (not shown), in which case the pusher wire 443, the pusher wire access point, and the attachment 445 may not be present. This delivery technique may function by enclosing the proximal basket end 407 within the closed cup biopsy forceps, while the remainder of the basket 400 remains distal to and on the exterior of the closed cup biopsy forceps. The basket 400 may then be released by opening the cup biopsy forceps. Utilization of the cup biopsy forceps for delivery may make the attachment 445 and the pusher wire 443 unnecessary.

The shape of the spherical region 418 may be generally spherical. However, the shape and/or size may vary, based on the shape and/or size of the aneurysm. In one example, the aneurysm basket 400 may be shaped, prior to deployment. In another example, the basket 400 may be flexible such that the shape of the basket will substantially conform to the shape of the aneurysm upon deployment of the basket 400. The ability of the basket 400 to conform to the shape and/or size of the aneurysm and the ability of the basket 400 to exert some amount of radial force on the aneurysm may ensure that the basket 400 is securely anchored within the aneurysm.

In one example, the length, shape and/or size of the neck region 415 may vary, depending on the length, shape and/or size of the aneurysm to be treated. In another example, the shape of the spherical region 418 may be generally spherical. However, the shape and/or size of the spherical region 418 may vary depending on the shape and/or size of the aneurysm to be treated. The basket 400 also may be flexible, such that the shape of the basket 400 will, to some degree, conform to the shape of the aneurysm.

The aneurysm basket 400, disclosed herein, may serve to anchor the patch 405 in the neck of the aneurysm. However, unlike microcoils, the basket 400 does not fill the aneurysm with a solid or semisolid mass. Instead, the basket 400 is hollow, relatively light, and pliable or flexible. Consequently, the basket 400 may function to occlude the aneurysm, while also reducing the occurrence of mass effect.

Movement of the basket 400 may be tracked by providing a radiopaque basket marker 450. The basket marker 450 may be located in a number of areas on the basket 400. In one example, the basket marker 450 may be located on the basket distal end 407.

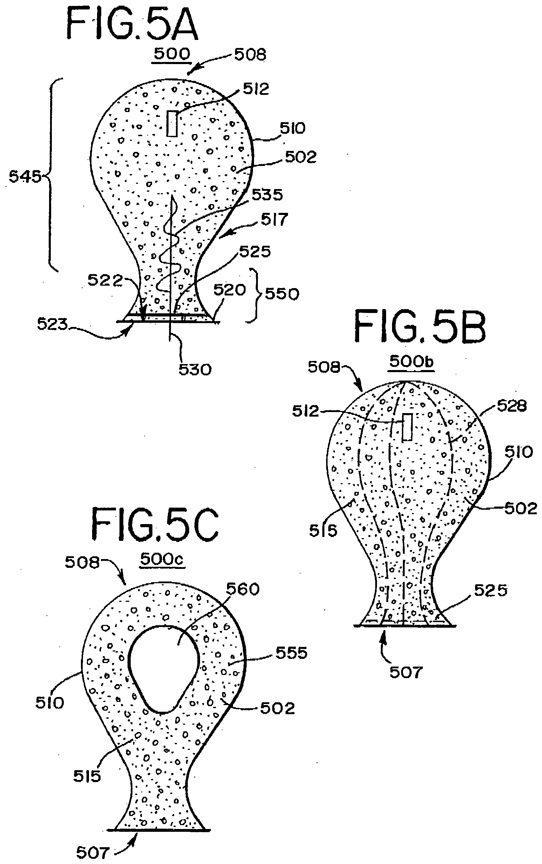

Fig. 5A illustrates a longitudinal cross-sectional view of an aneurysm insert 500a. The insert 500a may consist of a body 502 extending between a proximal insert end 507 and a distal insert end 508. The proximal insert end 507 and the distal insert end 508 may also be referred to as a proximal anchor end 507 and a distal anchor end 508, respectively. The body 502 may include an insert wall 510, which defines the exterior surface of the body 502. The insert 500 may also be fitted with a radiopaque marker 512, where the marker 512 may allow the location of the insert 500a to be determined when it is located within a patient. In one example, the marker 512 may be located within the body 502. In another example, the marker may be located on the insert wall 510.

The body 502 may be composed of a variety materials, including polymeric materials, shape memory alloys, stainless steel and collagenous biomaterials. In one example, the body 502 may be partially or entirely constructed from a compressible material 515. In another example, one or more portions of the body 502 may be substantially constructed from the compressible material 515. The compressible material 515 may be a polymeric material, such as a polyurethane. In one example, the compressible material 515 may be a polymeric material derived from polyvinyl alcohols, such as Ivalon®. In another example, the polymeric material may be porous Thoralon® or a derivative thereof. The compressible material 515 may also be a collagenous biomaterial, such as foam SIS. In either case, the compressible material 515 should be selected such that it can be compressed and loaded into a low profile catheter for delivery. The compressible material 515 should also be capable of self-expanding to occupy or contact the interior of the aneurysm, once the insert 500a has been deployed therein.

Porous THORALON can be formed by mixing the polyetherurethane urea (BPS-215), the surface modifying additive (SMA-300) and a particulate substance in a solvent. The particulate may be any of a variety of different particulates or pore forming agents, including inorganic salts. Preferably the particulate is insoluble in the solvent. The solvent may include dimethyl formamide (DMF), tetrahydrofuran (THF), dimethyacetamide (DMAC), dimethyl sulfoxide (DMSO), or mixtures thereof. The composition can contain from about 5 wt% to about 40 wt% polymer, and different levels of polymer within the range can be used to fine tune the viscosity needed for a given process. The composition can contain less than 5 wt% polymer for some spray application embodiments. The particulates can be mixed into the composition. For example, the mixing can be performed with a spinning blade mixer for about an hour under ambient pressure and in a temperature range of about 18 °C to about 27 °C. The entire composition can be cast as a sheet, or coated onto an article such as a mandrel or a mold. In one example, the composition can be dried to remove the solvent, and then the dried material can be soaked in distilled water to dissolve the particulates and leave pores in the material. In another example, the composition can be coagulated in a bath of distilled water. Since the polymer is insoluble in the water, it will rapidly solidify, trapping some or all of the particulates. The particulates can then dissolve from the polymer, leaving pores in the material. It may be desirable to use warm water for the extraction, for example water at a temperature of about 60 °C. The resulting pore diameter can also be substantially equal to the diameter of the salt grains.

The porous polymeric sheet can have a void-to-volume ratio from about 0.40 to about 0.90. Preferably the void-to-volume ratio is from about 0.65 to about 0.80. The resulting void-to-volume ratio can be substantially equal to the ratio of salt volume to the volume of the polymer plus the salt. Void-to-volume ratio is defined as the volume of the pores divided by the total volume of the polymeric layer including the volume of the pores. The void-to-volume ratio can be measured using the protocol described in AAMI (Association for the Advancement of Medical Instrumentation) VP20-1994, Cardiovascular Implants--Vascular Prosthesis section 8.2.1.2, Method for Gravimetric Determination of Porosity. The pores in the polymer can have an average pore diameter from about 1 micron to about 400 microns. Preferably the average pore diameter is from about 1 micron to about 100 microns, and more preferably is from about 1 micron to about 10 microns. The average pore diameter is measured based on images from a scanning electron microscope (SEM). Formation of porous THORALON is described, for example, in U.S. Pat. No. 6,752,826 and 2003/0149471 A1.

Dry foam SIS is extremely dense and compact, however when wetted this collagenous biomaterial expands substantially. Foam SIS consists of porous, 3-dimensional bodies formed from an SIS matrix. Foam SIS can be generated using a variety of techniques. For example, a general method of preparing foam SIS may involve enzymatically digesting a comminuted sample of SIS to provide a gelatinous solution of the collagenous biomaterial. The gelatinous solution may be added to a mold of the desired shape and submerged in a collagen crosslinking solution. Once the crosslinking is complete, a formed SIS foam may be provided. Next, the formed SIS foam may be removed from the crosslinking solution and lyophilized. The resulting product may be rinsed repeatedly with water and then compressed and subsequently lyophilized, providing the formed SIS foam in a dehydrated, highly dense, compacted, and flattened form.

In one example, the compressible material 515 may be a polymeric material that is entirely or partially coated with a lining of collagenous biomaterial, such as SIS, such that the insert wall 510 consists of the collagenous biomaterial. In a further example, the compressible material 515 may consist of an expandable polymeric material that is infused with a collagenous biomaterial (for example see U.S. Provisional Application Serial Nos. 60/558,794 and 60/558,667 filed March 31, 2004 (the benefit of which is claimed in U.S. Patent Application Publication No. 2005/0220848). In both cases, the presence of the collagenous biomaterial may serve as a scaffold or matrix for the replacement and repair of the damaged arterial tissue.

In one example, the proximal insert end 507 may include a patch 520. The patch 520 may comprise a variety of materials as are described for patch 105 in Fig. 1A. The patch 520 may be attached to the proximal insert end 507 using a variety of attachment techniques. These attachment techniques may include sutures, adhesive, heat sealing, "weaving" together, crosslinking, or other known methods of attachment. In a further example, the patch 520 may be attached to the proximal insert end 507 by a plurality of sutures. The patch 520 may include a patch distal side 522 and a patch proximal side 523. In one example, when the body 502 consists of foam SIS, the patch 520 may consist of a fabric or other flexible material, where the patch 520 serves to reinforce or provide structural support to the proximal insert end 507. Alternatively, the patch 520 may be integral with the foam SIS of the proximal insert end 507. In one example, when the body 502 consists of a compressible polymeric material, such as Ivalon, the patch 520 may consist of a collagenous biomaterial, such as SIS.

In one example, the patch 520 may be reinforced with a reinforcement material 525, where the reinforcement material 525 may serve to provide structural support. In addition, the reinforcement material 525 may be designed such that it is capable of being compressed when the insert 500 is contained within a microcatheter. In one example, the reinforcement material 525 may be applied to the surface of the patch 520, either on the patch distal side 522 or the patch proximal side 523 or both. In another example, the reinforcement material 525 may be situated within the patch 520. The reinforcement material 525 may be constructed from a variety of materials, including shape memory alloys, stainless steel, or polymeric materials. In one example, the reinforcement material 525 may be nitinol. In one example, the reinforcement material 525 may be woven or unwoven.

Whether the body 502 consists of a polymeric material or a collagenous biomaterial, the body 502 or a portion of the body 502 may incorporate a variety of materials, such as polymeric or ceramic materials, metal alloys, stainless steel or naturally derived collagenous materials. In one example, these materials may serve to reinforce the body 502. In another example, the body 502 may be interspersed with a plurality of wires 528. For example, Fig. 5B illustrates a longitudinal cross-sectional view of an aneurysm insert 500b that is interspersed with a plurality of wires 528. In one example, the wires 528 may be nitinol or stainless steel. For clarity, the pusher wire 530 and the corkscrew 535 are not shown in Fig. 5B.

A variety of delivery methods may be employed. In one example of a method, the aneurysm insert 500 may be delivered via a pusher wire 530, where the pusher wire 530 may be connected to the insert 500 by way of a corkscrew 535. The corkscrew 535 may be twisted into the insert 500 via the proximal insert end 507. The corkscrew 535 may allow the insert 500 to be selectively detached from the pusher wire 530 by untwisting or unscrewing the corkscrew 535. This attachment technique may also provide an ideal method of recapturing the insert 500 once it has been detached from the pusher wire 530. For example, once the insert 500 has been released, the corkscrew 535 may simply be screwed into any portion of the insert 500, thus recapturing the insert 500.

To provide an access point for the pusher wire 530, the patch 520 may have a pusher wire access point (see Fig. 1B, pusher wire access point 147).

In another example of a method, the insert 500 may be delivered using cup biopsy forceps (not shown), in which case the pusher wire access point may not be necessary. This delivery technique may function by enclosing the insert 500 within the closed cup biopsy forceps. The insert 500 may then be released by opening the cup biopsy forceps. In another example of a method, the insert 500 may be expelled from the delivery catheter using a pusher wire that is connected to the insert 500 by way of a selectively releasable lasso (not shown).

The shape and/or size of the insert 500 may vary based on a number of factors, including the size and shape of the aneurysm, the method of delivery, the material from which the insert 500 is constructed or other considerations. In one example, the insert 500 may resemble the general shape of an aneurysm. Thus the body 502 may have a spherical region 545 and a neck region 550, where the neck region 550 extends from the proximal insert end 507 to a distal neck end 517. The spherical region 545 may extend from the distal neck end 517 to the distal insert end 508. The spherical region 545 and the neck region 550 may be sized to snuggly occupy or contact the interior of the aneurysm. In one example, the insert 500 may adapt to the shape of the aneurysm upon deployment into the aneurysm and thus securely anchor the insert 500 within the aneurysm. Another factor in ensuring a secure fit between the insert 500 and the aneurysm may be to ensure that the insert 500 is oversized in relation to the aneurysm, such that the insert 500 may possess an amount of radial force after the insert 500 is deployed. In a further example, the body 502 and its constituent parts may be shaped and/or sized prior to deployment, such that the insert 500 will fit securely within a specific aneurysm.

In one example, the body 502 may include an interior region 555 and a void or open space 560. For example, Fig. 5C illustrates a longitudinal cross-sectional view of an aneurysm insert 500c, where the body 502 includes the interior region 555 and the void or open space 560. The interior region may be partially or entirely composed of the compressible material 515. In addition, the interior region may be reinforced with a variety of materials, such as polymeric or ceramic materials, metal alloys, stainless steel or collagenous biomaterials. The void or open space 560 may consist of air, an inert gas or a fluid. In one example, the void 560 may fill with body fluids following deployment of the insert 500b.

In addition, the aneurysm insert 500, disclosed herein, may serve to anchor the patch 520 in the neck of the aneurysm. Thus the insert 500 may function to occlude the aneurysm. Furthermore, when the insert 500 partially or entirely comprises a collagenous biomaterial, it may serve as a scaffold or matrix for the replacement and repair of the damages arterial tissue of the aneurysm.

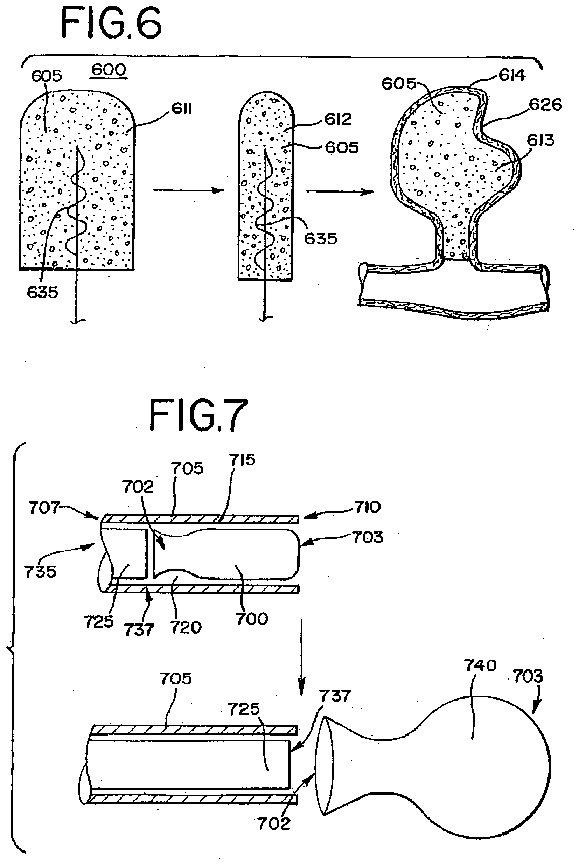

Fig. 6 illustrates a longitudinal cross-sectional view of an aneurysm insert 600, where the insert 600 has an expanded conformation 611, a compressed conformation 612 and a deployed conformation 613. In one example, the expanded conformation 611 may be generally cylindrical in shape (as shown). In another example, the expanded conformation 611 may be shaped, prior to deployment, to resemble an aneurysm. The expanded conformation 611 may be compressed to provide the compressed conformation 612. The compressed conformation 612 may exist when the insert 600 is contained within a low profile delivery catheter (not shown). Once the insert 600 is deployed into an aneurysm 614, the compressed conformation 612 may expand to provide the deployed conformation 613, which may substantially occupy or contact the interior of the aneurysm 614. Thus the deployed conformation 613 may generally adapt to the shape and/or size of the aneurysm 614. An area 640 represents the space previously occupied by the expanded conformation 611 before it was deployed in the aneurysm 614. For example, the deployed conformation 613 may adapt to an aneurysm irregularity 626. The size of the insert 600 may be selected based on the size of the aneurysm 614. Ideally, the insert 600 may be sized in relation to the aneurysm 620, such that the insert 600 will expand enough to securely anchor the insert 600 within the aneurysm 614. As discussed previously, the insert 600 may be delivered using a variety of techniques, such as a corkscrew 635. Furthermore, the insert 600 may be partially or entirely composed of a compressible material 605, which may be a polymeric material or a collagenous biomaterial.

Fig. 7 illustrates a longitudinal cross-sectional view of compressed aneurysm insert 700 that is loaded in a microcatheter 705 and an expanded aneurysm insert 740.

A variety of delivery techniques may be utilized to deploy the insert 700. One such technique may employ a microcatheter 705, as described herein. In this configuration, the insert 700 may extend between a proximal insert end 702 and a distal insert end 703. The microcatheter 705 may extend between a proximal microcatheter end 707 and a distal microcatheter end 710. Furthermore, the microcatheter 705 may have a microcatheter wall 715 defining a lumen 720 therein. The lumen 720 may contain a pusher member 725, which may have a pusher member proximal end 735 and a pusher member distal end 737. The insert 700 may be located within the catheter 705 such that it is distal to the pusher member 725, but located substantially within the catheter 705.

In this configuration, deploying the insert 700 may be achieved by manipulating the pusher member 725, such that it travels in the distal direction in relation to the microcatheter wall 715. Deployment of the insert 700, resulting in an expanded aneurysm insert 740, may be accomplished when the pusher member distal end 740 reaches the microcatheter distal end 710. Utilization of the pusher member 725 for deployment of the insert 700 may obviate the need for the pusher wirer, as previously described.

Fig. 8 illustrates an aneurysm basket or sealing device 1010 percutaneously deployed through a dilatation area 1012 of a body vessel 1014 formed by an aneurysm 1016. As shown, aneurysm 1016 is formed of a sac or dome 1018 having a neck 1019 extending therefrom to define the dilatation area 1012. As known, an aneurysm is formed by a weakened or dilatation area on a body vessel. The dilatation area may be congenital or caused by high blood pressure. Blood pressure causes the dilatation area to dilate and expand to form an abnormal dome or sac-like structure of the body vessel.

The sealing device 1010 is configured to be compressed or collapsed in a loaded state for delivery (and re-positioning) of the sealing device 1010 to a deployment location within the body vessel 1014. At the deployment location, the sealing device 1010 is configured to self-expand in an expanded state within the body vessel 1014. As mentioned in greater detail below, embodiments of a delivery apparatus are configured to allow the sealing device 1010 to be partially deployed in the aneurysm. Via fluoroscopy, an assessment may then be made by the practitioner as to the placement of the sealing device, and adjustments or re-positioning of the sealing device relative to the aneurysm may be made as deemed necessary.

Fig. 9 depicts the sealing device 1010 comprising an anchor member 1020 having a neck portion 1021 and extending to a sealing lip 1022. In the expanded state, the anchor member 1020 takes on a shape consistent with the dome 1018 of the aneurysm 1016 to be introduced and disposed therein. When disposed in the aneurysm 1016, the anchor member 1020 allows the sealing device 1010 to maintain placement at the dilatation area 1012 for treatment of the aneurysm 1016.

In this embodiment, the anchor member 1020 is a woven basket made of super-elastic material and/or a shape-memory alloy, such as nitinol. In this embodiment, the anchor member 1020 is woven in a mandrel and is manufactured to be compressed in a loaded state for delivery and self-expandable in an expanded state for deployment. For example, this can be accomplished with a shape-memory alloy (e.g. nitinol) being formed in a rigid state (e.g., an austenite state of the nitinol) and a flexible state (e.g. a martensitic state). The anchor member may be woven with shape-memory alloy by any suitable means known in the art.

The sealing device 1010 further includes a base or patch 1030 attached to the sealing lip 1022. The patch 1030 may comprise a variety of materials as are described for patch 105 in Fig. 1A. When the anchor member 1020 is deployed in the sac 1018 of the aneurysm 1016, the base 1030 of the device 1010 is preferably placed in contact with the body vessel 1014 at the dilatation area 1012 whereat the aneurysm had originated.

In one embodiment, the base 1030 may comprise a collagenous biomaterial or connective tissue 1036 for tissue repair of the body vessel 1014 at the dilatation area 1012. Preferably, the base 1030 of the device 1010 is placed in contact with the body vessel 1014 and disposed at the dilatation area 1012 to seal the aneurysm 1016. Subsequently, as the connective tissue 1036 contacts the body vessel 1014 at the dilatation area1012, the connective tissue 1036 may induce tissue growth around the dilatation area 1012, wherein host cells of the body vessel 1014 may become stimulated to proliferate and differentiate into site-specific connective tissue structures. The connective tissue 1036 may then be substantially replaced, and resulting in tissue remodeling, sealing the dilatation area 1012. In a further embodiment, the base 1030 may extend beyond the sealing lip 1022 and may overlap or cover the neck portion 1021.

In Fig. 10 the base 1030 includes struts 1032 attached to the sealing lip 1022 and extend inwardly therefrom to a common point 1034 for collapsing the sealing device 1010. As mentioned above, the sealing device 1010 is configured to be compressible for loading in a delivery apparatus and self-expandable for deployment in the body vessel 1014. This may be accomplished by any suitable means, such as by employing shape-memory alloys (e.g. Nitinol). As shown in Fig. 10, the struts 1032 extend radially inward from the sealing lip 1022 to common point 1034. Each of struts 1032 may be attached to the sealing lip 1022 and each other by any suitable means. For example, the super-elastic material of the anchor member 1020 may be woven to each of the struts 1032 to allow pivotal movement at the sealing lip 1022. Additionally, the struts 1032 may be crimped together at the common point 1034, allowing for pivotal movement between the loaded and expanded states.

In this embodiment, the base 1030 has a collagenous biomaterial or connective tissue 1036 disposed on the struts 1032 for tissue repair of the body vessel 1014 at the dilatation area 1012. Preferably, the base 1030 of the device 1010 is placed in contact with the body vessel 1014 and disposed at the dilatation area 1012 to seal the aneurysm 1016. Subsequently, as the connective tissue 1036 contacts the the body vessel 1014 at the dilatation area 1012, the connective tissue 1036 may induce tissue growth around the dilatation area 1012, wherein host cells of the body vessel 1014 may become stimulated to proliferate and differentiate into site-specific connective tissue structures. The connective tissue 1036 may then be substantially replaced, and resulting in tissue remodeling, sealing the dilatation area 1012.

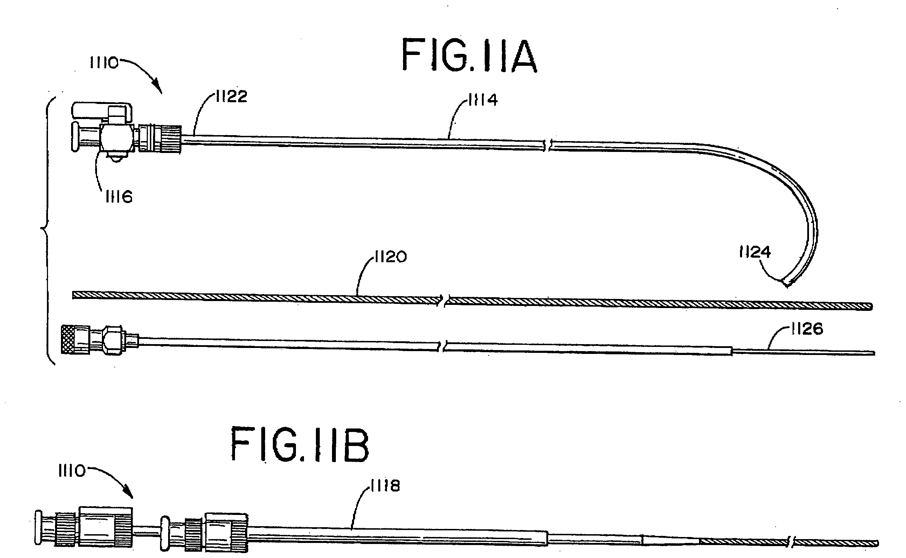

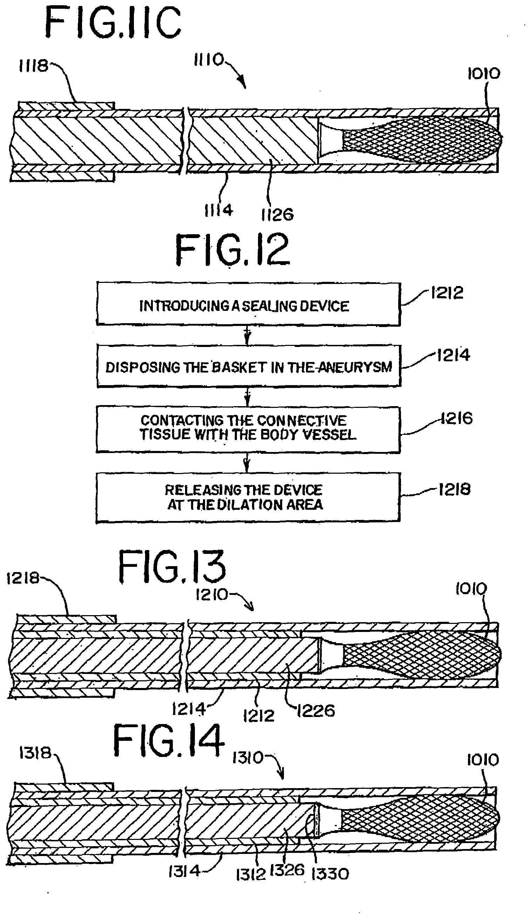

Figures 11A - 11C depict an example of aneurysm sealing apparatus 1110 which implements the aneurysm sealing device 1010 in accordance with one embodiment of the present invention. As shown, the apparatus 1110 includes a catheter 1114 defining a catheter lumen and preferably made from a soft, flexible material such as silicone or any other suitable material. Generally, the catheter 1114 has a proximal end 1122, a distal end 1124, and a plastic adapter or hub 1116 to receive the sealing device 1010 to be advanced therethrough. The size of the catheter 1114 is based on the size of the body vessel having the aneurysm. The apparatus 1110 further includes a wire guide 1120 which provides the catheter 1114 a path during insertion within a body vessel. The size of the wire guide 1120 is based on the inside diameter of the catheter 1114.

The, the apparatus 1110 further includes a polytetrafluoroethylene (PTFE) introducer sheath 1118 for percutaneously introducing the catheter 1114 in a body vessel. Of course, any other suitable material may be used without falling beyond the scope or spirit of the present invention. The introducer sheath 1118 may have a size of about 3-French to 8-French and allows the catheter 1114 to be inserted therethrough to a desired location in the body vessel. The introducer sheath 1118 receives the catheter 1114 and provides stability of the catheter 1114 at a desired location of the body vessel. For example, the introducer sheath 1118 may stay stationary within a common visceral artery, and adds stability to the catheter 1114 as the catheter is advanced through the introducer sheath to the dilatation area in the vasculature.

When the distal end 1124 of the catheter 1114 is at the dilatation area in the body vessel, the sealing device is loaded at the proximal end 1122 of the catheter 1114 and is advanced through the catheter for deployment through the distal end 1124. In this example, a push wire 1126 is used to mechanically advance or push the sealing device through the catheter 1114. The size of the push wire 1126 depends on the diameter of the lumen as defined by the catheter 1114.

Fig. 12 illustrates an example of one method 1210 of sealing a dilatation area of a body vessel formed by an aneurysm. In this example, the introducer sheath is percutaneously introduced into the body vessel of a patient and the catheter is passed through the introducer sheath to position the catheter at the dilatation area in the body vessel. The sealing device, which is compressed to its loaded state, is loaded in the hub at the proximal end of the catheter. In step 1212, the device is advanced by the pusher wire to be introduced in the body vessel for sealing the dilatation area in accordance with this method.

In step 1214, a first portion of the sealing device is deployed (e.g., the basket) in the sac of the aneurysm as a remaining portion (e.g., the neck and base of the sealing device) is held in the catheter.

In step 1216, the location of the first portion in the sac of the aneurysm is identified by any suitable means, such as by fluoroscopy, relative to the body vessel to confirm that the connective tissue is contacting the body vessel. Preferably, the base having the connective tissue is placed at the dilatation area such that the connective tissues contact the body vessel to seal the dilatation area.

However, if it is determined via fluoroscopy in step 1216 that the first portion of the sealing device is not at the dilatation area or at a desired location within the body vessel, then the position of the catheter is moved fore or aft relative to the body vessel such that the first portion is placed at the desired point of sealing. At this point, the sealing device is still able to be compressed in the catheter in the event that it is deemed that the device should be retrieved from the body vessel or repositioned therein.

If the first portion has been placed at a desired point of insertion within the sac, then the remaining portion of the device is advanced and released. This completes the deployment in step 1218 of the sealing device in the body vessel for tissue repair.

Preferably, the first portion is deployed in the body vessel by proximally moving the distal end of the catheter against the first portion. The remaining portion is then deployed at the dilatation area by moving the catheter distally. As the remaining portion is being deployed, the distal end of the catheter is moved back.

Fig. 13 illustrates an example of sealing apparatus 1210. The apparatus 1210 comprises an inner catheter 1212 in which the sealing device is loaded, and outer catheter 1214 housing the inner catheter 1212, and an introducer sheath 1218 for percutaneous insertion of the catheters 1212 and 1214. As shown, the inner catheter 1212 houses a loaded sealing device. As the first portion of the sealing device is partially deployed, the outer catheter maintains the sealing device in a partially expanded state within its lumen. When desired, retraction of the outer catheter from the deployment location allows the sealing device to be deployed in its expanded state.

Fig. 14 depicts another example of an apparatus 1310 for sealing a dilatation area of a body vessel formed by an aneurysm. In this example, apparatus 1310 used herein includes an electrolytic mechanism for detachment of the sealing device 1010. As shown, the sealing device 1010 is proximally attached to the distal end of a push wire 1326 by a connection joint 1330, e.g. a soldering joint, and is advanced through inner catheter 1312 to the dilatation area in a body vessel of a patient. The sealing device 1010 is then advanced through the inner catheter 1312 by the push wire 1326 to the dilatation area for treatment of the aneurysm. Upon deployment, an electrical current may be passed through the connection joint 1330 to disengage or detach the sealing device 1010 from the apparatus 1310. This may be accomplished by any suitable system or mechanism known in the electrical arts. In this example, the connection joint is a solder joint wherein the electric current results in dissolution of the solder, thereby detaching the sealing device from the apparatus.

In another example, the sealing device 1010 may be delivered using cup biopsy forceps (not shown). The delivery of the sealing device may function by enclosing the basket thereof within a set of closed cup biopsy forceps, while the remainder of the device remains distal to and on the exterior of the closed cup biopsy forceps. The basket may then be released by opening the cup biopsy forceps.

It is to be understood that the sealing apparatuses 1110, 1210, and 1310 are merely examples of an apparatus that may be used to deploy the sealing device in a body vessel. Of course, other apparatuses, assemblies, and systems may be used to deploy any embodiment of the sealing device without falling beyond the scope of the present invention.

It is therefore intended that the foregoing detailed description be regarded as illustrative rather than limiting, and that it be understood that it is the following claims, including all equivalents, that are intended to define the scope of this invention.

## Claims

1. An aneurysm insert (1010) adapted for intravascular delivery to the interior of an aneurysm, the insert (1010) comprising a structure (1020) that in a rest state intrinsically defines an exterior spheroidal surface, the structure (1020) being compressible to enable passage through a neck of the aneurysm;
in which said structure is adapted through shape memory to return to said rest state after compression;
wherein said structure (1020) is an aneurysm basket formed of a plurality of wires that includes a basket wall extending between a proximal basket end and a distal basket end, where the basket wall encloses a cavity and the proximal basket end comprises a ring, the aneurysm basket further comprising a basket neck region (1021) extending between the proximal basket end and a distal neck end and a spherical region extending between the distal neck end and the distal basket end.; wherein the aneurysm insert (1010) further comprises a patch (1030) circumscribed by and attached to the ring and adapted substantially to seal the neck of the aneurysm, said structure (1020) serving in use to anchor said patch (1030); and said patch (1030) being separated from the spheroidal surface by said neck region (1021).

2. An insert according to Claim 1, wherein the patch (1030) is formed of collagenous biomaterial or connective tissue.

3. An insert according to Claim 2, wherein the patch (1030) is configured in use to induce tissue growth.

4. An insert according to any one of the preceding claims, wherein said neck region (1021) defined by the structure (1020) extends to a sealing lip (1022), with said patch (1030) being attached to said sealing lip (1022).

5. An insert according to Claim 1, wherein said wire basket comprises two mutually orthogonal wire paths.

6. An insert according to any one of the preceding claims, wherein the structure (1020) is adapted to permit the exterior spheroidal surface to adapt to deviations from sphericity in the interior surface of the aneurysm.

7. An insert according to any one of the preceding claims, where the bulk density of said structure (1020), being the mass of the structure (1020) divided by the volume enclosed by said spheroidal surface, is less than blood density.

## Patentansprüche

1. Aneurysma-Einsatz (1010), der zur intravaskulären Freisetzung an das Innere eines Aneurysmas ausgelegt ist, wobei der Einsatz (1010) eine Struktur (1020) umfasst, die in einem Ruhezustand intrinsisch eine äußere kugelige Oberfläche definiert, wobei die Struktur (1020) zusammenpressbar ist, damit sie durch einen Hals des Aneurysmas geführt werden kann;
wobei die Struktur durch Formgedächtnis dazu ausgelegt ist, nach der Kompression wieder in den Ruhezustand zurückzukehren;
wobei die Struktur (1020) ein Aneurysmakörbchen ist, das aus einer Vielzahl von Drähten ausgebildet ist und eine Körbchenwand aufweist, die sich zwischen einem proximalen Körbchenende und einem distalen Körbchenende erstreckt, wobei die Körbchenwand einen Hohlraum umschließt und das proximale Körbchenende einen Ring umfasst, wobei das Aneurysmakörbchen ferner eine Körbchenhalsregion (1021) umfasst, die sich zwischen dem proximalen Körbchenende und einem distalen Körbchenende erstreckt, sowie eine kugelförmige Region, die sich zwischen dem distalen Halsende und dem distalen Körbchenende erstreckt;
wobei der Aneurysmaeinsatz (1010) ferner ein Patch (1030) umfasst, das vom Ring umschrieben wird und daran befestigt ist und das dazu ausgelegt ist, den Hals des Aneurysmas im Wesentlichen dicht zu verschließen, wobei die Struktur (1020) bei der Verwendung zur Verankerung des Patchs (1030) dient; und wobei das Patch (1030) von der kugelförmigen Oberfläche durch die Halsregion (1021) getrennt ist.

2. Einsatz nach Anspruch 1, wobei das Patch (1030) aus kollagenem Biomaterial oder Bindegewebe besteht.

3. Einsatz nach Anspruch 2, wobei das Patch (1030) dazu ausgelegt ist, bei Verwendung Einwachsen von Gewebe auszulösen.

4. Einsatz nach einem der vorhergehenden Ansprüche, wobei sich die Halsregion (1021), die von der Struktur (1020) definiert wird, bis zu einer Dichtungslippe (1022) erstreckt, wobei das Patch (1030) an der Dichtungslippe (1022) befestigt ist.

5. Einsatz nach Anspruch 1, wobei das Drahtkörbchen zwei gegenseitig orthogonale Drahtwege umfasst.

6. Einsatz nach einem der vorhergehenden Ansprüche, wobei die Struktur (1020) dazu ausgelegt ist, es der äußeren kugelförmigen Oberfläche zu gestatten, sich an Abweichungen von der Sphärizität in der inneren Oberfläche des Aneurysmas anzupassen.

7. Einsatz nach einem der vorhergehenden Ansprüche, wobei die Raumdichte der Struktur (1020), bei der es sich um die Masse der Struktur (1020) dividiert durch das von der kugelförmigen Oberfläche umschlossene Volumen handelt, geringer als die Blutdichte ist.

## Revendications

1. Insert pour anévrisme (1010), prévu pour être installé par voie intravasculaire à l'intérieur d'un anévrisme, l'insert (1010) comprenant une structure (1020) qui, dans un état de repos, définit de manière intrinsèque une surface extérieure sphéroïdale, la structure (1020) étant compressible de manière à permettre le passage à travers le col de l'anévrisme ;
ladite structure étant prévue, par mémoire de forme, pour revenir dans ledit état de repos après la compression ;
ladite structure (1020) étant un panier d'anévrisme formé d'une pluralité de fils, qui comprend une paroi de panier s'étendant entre une extrémité de panier proximale et une extrémité de panier distale, la paroi de panier renfermant une cavité et l'extrémité de panier proximale comprenant une bague, le panier d'anévrisme comprenant en outre une région de col de panier (1021) s'étendant entre l'extrémité de panier proximale et une extrémité de col distale et une région sphérique s'étendant entre l'extrémité de col distale et l'extrémité de panier distale ;
l'insert pour anévrisme (1010) comprenant en outre une pastille (1030) circonscrite par la bague et attachée à celle-ci et prévue pour sceller sensiblement le col de l'anévrisme, ladite structure (1020) servant, pendant l'utilisation, à ancrer ladite pastille (1030) ; et ladite pastille (1030) étant séparée de la surface sphéroïdale par ladite région de col (1021).

2. Insert selon la revendication 1, dans lequel la pastille (1030) est formée de biomatériau collagène ou de tissu conjonctif.

3. Insert selon la revendication 2, dans lequel la pastille (1030) est configurée, pendant l'utilisation, pour induire une croissance tissulaire.

4. Insert selon l'une quelconque des revendications précédentes, dans lequel ladite région de col (1021) définie par la structure (1020) s'étend jusqu'à une lèvre de scellement (1022), ladite pastille (1030) étant attachée à ladite lèvre de scellement (1022).

5. Insert selon la revendication 1, dans lequel ledit panier en fil comprend deux chemins de fil perpendiculaires l'un à l'autre.

6. Insert selon l'une quelconque des revendications précédentes, dans lequel la structure (1020) est prévue pour permettre à la surface extérieure sphéroïdale de s'adapter à des défauts de sphéricité dans la surface intérieure de l'anévrisme.

7. Insert selon l'une quelconque des revendications précédentes, dans lequel la densité en vrac de ladite structure (1020), étant la masse de la structure (1020) divisée par le volume renfermé par ladite surface sphéroidale, est inférieure à la densité du sang.
